(19) Europäisches Patentamt
European Patent Office
Office européen des brevets

(11) EP 3 829 511 B1

(12) EUROPEAN PATENT SPECIFICATION

(45) Date of publication and mention of the grant of the patent:
**14.02.2024 Bulletin 2024/07**

(21) Application number: **19749613.6**

(22) Date of filing: **23.07.2019**

(51) International Patent Classification (IPC):
***A61F 13/534*** *(2006.01)* ***A61L 15/42*** *(2006.01)*
***A61L 15/60*** *(2006.01)* ***C08F 220/06*** *(2006.01)*

(52) Cooperative Patent Classification (CPC):
**A61F 13/534; A61L 15/42; A61L 15/60; C08F 220/06**

(86) International application number:
**PCT/EP2019/069833**

(87) International publication number:
**WO 2020/025401 (06.02.2020 Gazette 2020/06)**

(54) **FLUID-ABSORBENT CORE**

FLÜSSIGKEITSABSORBIERENDER KERN

NOYAU ABSORBANT LES FLUIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2018 PCT/CN2018/098037**

(43) Date of publication of application:
**09.06.2021 Bulletin 2021/23**

(73) Proprietor: **BASF SE**
**67056 Ludwigshafen am Rhein (DE)**

(72) Inventors:
- **GE, Hui**
  **Shanghai, 200137 (CN)**
- **HERFERT, Norbert**
  **Shanghai, 200137 (CN)**
- **ELLIOTT, Mark**
  **67056 Ludwigshafen (DE)**
- **MITCHELL, Michael**
  **Charlotte, North Carolina 28273 (US)**
- **DUAN, Li Guo**
  **Shanghai, 200137 (CN)**

(74) Representative: **BASF IP Association**
**BASF SE**
**GBI-C006**
**67056 Ludwigshafen (DE)**

(56) References cited:
**EP-A1- 2 565 031**
**EP-A1- 2 944 376**
**US-A1- 2017 281 425**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to a fluid-absorbent core (80) comprising at least two layers, an upper layer (91) and a lower layer (92), each layer comprising from 0 to 10% by weight fibrous material and from 90 to 100% by weight water-absorbent polymer particles, based on the sum of water-absorbent polymer particles and fibrous material, wherein within the upper layer (91) a water absorbent mixture comprising at least 10% by weight of a first type of water-absorbent polymer particles, water-absorbent polymer particles G, with a vortex of less than 30s and at least 50% by weight of a second type of water-absorbent polymer particles, water-absorbent polymer particles H, with a CRC of at least 25 g/g wherein water-absorbent polymer particles G are obtained by agglomerating fine water-absorbing polymer particles by using a solution or suspension comprising

a) 0,04 to 1,2 % by weight water-soluble or water-dispersible polymeric binders or a mixture thereof, based on the water-absorbent polymer particles,
b) 20 to 70% by weight of water based on the water-absorbent polymer particles, and
c) 5 to 20% by weight of a water-miscible organic solvent based on the water-absorbent polymer particles

and wherein fine water-absorbing polymer particles having an average particle diameter of not larger than 300 μm and a fluid-absorbent article comprising said fluid-absorbent core (80).

The production of fluid-absorbent articles is described in the monograph "Modern Superabsorbent Polymer Technology", F.L. Buchholz and A.T. Graham, Wiley-VCH, 1998, pages 252 to 258.

The currently commercially available disposable diapers consist typically of a liquid-pervious topsheet (89), a liquid-impervious backsheet (83), a water-absorbing storage layer (absorbent core) (80) between layers (89) and (83), and an acquisition distribution layer (70) between layers (89) and (80).

Usually the several layers of fluid-absorbent articles fulfill definite functions such as dryness for the upper liquid-pervious layer, vapor permeability without wetting through for the lower liquid-impervious layer, a flexible, vapor permeable and fluid-absorbent core, showing fast absorption rates and being able to retain quantities of body fluids and an acquisition-distribution layer between the upper layer and the core, acting as transport and distribution layer of the discharged body fluids.

The preparation of water-absorbing polymer particles is generally described in the monograph "Modern Superabsorbent Polymer Technology", F.L. Buchholz and A.T. Graham, Wiley-VCH, 1998, pages 71 to 103. The water-absorbing polymer particles are also referred to as "fluid-absorbing polymer particles", "superabsorbent polymers" or "superabsorbents".

Various types and grades of superabsorbents are available, e.g. superabsorbents that have been produced by

- gel polymerisation in the batch process or tubular reactor and subsequent comminution in meat grinder, extruder or kneader, as described for example in EP 445 619 A2 and DE 19 846 413 A1;
- polymerisation in kneader with continuous comminution by contrarotatory stirring shafts for example, as described for example in WO 01/38 402 A1;
- polymerisation on belt and subsequent comminution in meat grinder, extruder or kneader, as described for example in EP 955 086 A2, DE 38 25 366 A1 or US 6,241,928
- emulsion polymerisation, which produces bead polymers having a relatively narrow gel size distribution, as described for example in EP 457 660 A1;
- polymerizing droplets of the monomer in a surrounding heated gas phase, for example using a system described in WO 2008/040715 A2, WO 2008/052971 A1, WO 2008/069639 A1 and WO 2008/086976 A1, WO 2014/079694, WO 2015/028327, WO 2015/028158
- that have been produced by agglomerating water-absorbent particles with a mean particle size of less than 150 μm, as for example disclosed in EP2 944 376 A1.

In the last years, there has been a trend toward very thin disposable diapers. To produce thin disposable diapers, the proportion of cellulose fibers in the water-absorbing storage layer has been lowered or is almost missing.

A core-structure for ultrathin fluid-absorbent products can be formed from absorbent paper. Such structures are for example described in WO2011/086842, EP 2 565 031 A1,

EP 2 668 936 A1. But the known ultrathin fluid-absorbent products comprising absorbent paper structures have deficiencies in respect to fluid acquisition, leakage and rewet properties.

US2017/0281425A1 discloses core structures for thin fluid-absorbent products comprising absorbent particles obtained by polymerizing droplets of a monomer solution in a surrounding heated gas phase.

To prevent leakage and wet feeling it is preferred to have thicker acquisition-distribution layers so that the time to absorb the body fluid is preferably short. But this contravenes the trend to thinner absorbent articles, as the thickness

is also a great issue in respect to absorbent articles especially in respect to noticeability for adult articles and also hindrance, especially for baby diapers and pants.

It is therefore an object of the present invention to provide a fluid-absorbent core for fluid-absorbent products with an improved performance.

It is also an object of the present invention to provide absorbent cores with improved fluid storage capacity to avoid leakage.

It is furthermore an object of the present invention to provide absorbent cores with improved rewet performance.

It is also an object of the present invention to provide absorbent articles with improved core structures.

The object is achieved by a fluid-absorbent core (80) comprising at least two layers, an upper layer (91) and a lower layer (92), each layer comprising from 0 to 10% by weight fibrous material and from 90 to 100% by weight water-absorbent polymer particles, based on the sum of water-absorbent polymer particles and fibrous material, wherein within the upper layer (91) a water absorbent mixture comprising at least 10% by weight of a first type of water-absorbent polymer particles, water-absorbent polymer particles G, with a vortex of less than 30s and at least 50% by weight of a second type of water-absorbent polymer particles, water-absorbent polymer particles H, with a CRC of at least 25 g/g, wherein water-absorbent polymer particles G are obtained by agglomerating fine water-absorbing polymer particles by using a solution or suspension comprising

d) 0,04 to 1,2 % by weight water-soluble or water-dispersible polymeric binders or a mixture thereof, based on the water-absorbent polymer particles,
e) 20 to 70% by weight of water based on the water-absorbent polymer particles, and
f) 5 to 20% by weight of a water-miscible organic solvent based on the water-absorbent polymer particles

and wherein fine water-absorbing polymer particles having an average particle diameter of not larger than 300 μm.

In another embodiment according to the invention the water-absorbent polymer particles G have a vortex of less than 25s, preferably have a vortex of less than 20s more preferably the water-absorbent polymer particles G have a vortex of less than 15s, most preferably the water-absorbent polymer particles G have a vortex of less than 10s.

In a further embodiment the water-absorbent polymer particles H have a CRC of at least 30 g/g, preferably the water-absorbent polymer particles H have a CRC of at least 35 g/g.

According to the present invention it is preferred that the water-absorbent polymer particles G and H of the fluid absorbent core are irregular particles.

The water-absorbent polymer particles G of the fluid-absorbent core (80) according to the invention are produced by agglomeration of fine water-absorbing polymer particles.

Whereas according to the invention the fine water-absorbent polymer particles having an average particle diameter of not larger than 300 μm. The fine water-absorbent polymer particles according to the invention preferably having an average particle diameter of at maximum 150 μm, more preferably at maximum 120 μm.

The water-absorbent polymer particles G are obtainable by agglomerating non-surface post-crosslinked fine water-absorbent polymer particles, surface post-crosslinked fine water-absorbent polymer particles or a blend of non-surface post-crosslinked fine water-absorbent polymer particles and surface post-crosslinked fine water-absorbent polymer particles, drying, grinding, sieving and classification of the agglomerated water-absorbent polymer particles.

One preferred fluid-absorbent core (80) according to the invention comprises within the lower layer (92) water-absorbent polymer particles with a CRC of at least 30g/g and a vortex of at least 30 s.

The object is furthermore achieved by a fluid absorbent article, comprising

(A) an upper liquid-pervious sheet (89),
(B) a lower liquid-impervious sheet (83),
(C) a fluid-absorbent core (80) comprising at least two layers, an upper layer (91) and a lower layer (92), each layer comprising from 0 to 10% by weight fibrous material and from 90 to 100% by weight water-absorbent polymer particles, based on the sum of water-absorbent polymer particles and fibrous material,

wherein within the upper layer (91) are a water absorbent mixture comprising at least 10% by weight of a first type of water-absorbent polymer particles, water-absorbent polymer particles G, with a vortex of less than 30s and at least 50% by weight of a second type of water-absorbent polymer particles, water-absorbent polymer particles H, with a CRC of at least 25 g/g, wherein water-absorbent polymer particles G are obtained by agglomerating fine water-absorbing polymer particles by using a solution or suspension comprising

a) 0,04 to 1,2 % by weight water-soluble or water-dispersible polymeric binders or a mixture thereof, based on the water-absorbent polymer particles,
b) 20 to 70% by weight of water based on the water-absorbent polymer particles, and

c) 5 to 20% by weight of a water-miscible organic solvent based on the water-absorbent polymer particles

and wherein fine water-absorbing polymer particles having an average particle diameter of not larger than 300 μm.

(D) an optional acquisition-distribution layer (70) between the upper liquid-pervious sheet (89) and the fluid-absorbent core (80),
(F) other optional components.

Furthermore according to another embodiment the fluid absorbent article has a water pouring time of less than 35 s and a rewet value of less than 0,8 g.
The inventive fluid absorbent article preferably has a TAC $_{AP,30\ min}$ of more than 500 g/g.
Water-absorbent polymer particles, e.g. water-absorbent polymer particles H and the water-absorbent polymer particles of the lower layer (92) and fine water-absorbent polymer particles, are generally produced by a process, comprising the steps forming water-absorbent polymer particles by polymerizing a monomer solution, comprising

a) at least one ethylenically unsaturated monomer which bears acid groups and may be at least partly neutralized,
b) optionally one or more crosslinker,
c) at least one initiator
d) optionally one or more ethylenically unsaturated monomers copolymerizable with the monomers mentioned under a),
e) optionally one or more water-soluble polymers, and
f) water,

optionally coating of water-absorbent polymer particles with at least one surface-postcrosslinker and thermal surface-postcrosslinking of the coated water-absorbent polymer particles
drying, optionally grinding, sieving and classification of the water-absorbent polymer particles.

Fine water-absorbent polymer particles are removed from the superabsorbent production. Fine water-absorbent polymer particles are undesirable as they lead to a reduced product performance e.g. by supporting gel blocking.

Detailed description of the invention

A. Definitions

As used herein, the term "fluid-absorbent article" refers to any three-dimensional solid material being able to acquire and store fluids discharged from the body. Preferred fluid-absorbent articles are disposable fluid-absorbent articles that are designed to be worn in contact with the body of a user such as disposable fluid-absorbent pantyliners, sanitary napkins, catamenials, incontinence inserts/pads, diapers, training pant diapers, breast pads, interlabial inserts/pads or other articles useful for absorbing body fluids.
As used herein, the term "fluid-absorbent composition" refers to a component of the fluid-absorbent article which is primarily responsible for the fluid handling of the fluid-absorbent article including acquisition, transport, distribution and storage of body fluids.
As used herein, the term "fluid-absorbent core", "absorbent core" or "absorbent paper" refers to a fluid-absorbent composition comprising at least two layers of water-absorbent polymer particles and optionally fibrous material, nonwoven material and tissue material and optionally adhesive. The fluid-absorbent core is primarily responsible for the fluid handling of the fluid-absorbent article including acquisition, transport, distribution and storage of body fluids.
As used herein, the term "layer" refers to a fluid-absorbent composition whose primary dimension is along its length and width. Thus a layer can comprise laminates, composites, combinations of several sheets or webs of different materials.
As used herein the term "x-dimension" refers to the length, and the term "y-dimension" refers to the width of the fluid-absorbent composition, layer, core or article. Generally, the term "x-y-dimension" refers to the plane, orthogonal to the height or thickness of the fluid-absorbent composition, layer, core or article.
As used herein the term "z-dimension" refers to the dimension orthogonal to the length and width of the fluid absorbent composition, layer, core or article. Generally, the term "z-dimension" refers to the height of the fluid-absorbent composition, layer, core or article.
As used herein, the term "basis weight" indicates the weight of the fluid-absorbent core or any tissue per square meter and does not include the chassis of the fluid-absorbent article. The basis weight is determined at least at two

different regions of the fluid-absorbent core or any tissue respectively and is taken as the average of the at least two results.

Further, it should be understood, that the term "upper" refers to fluid-absorbent composition which are nearer to the wearer of the fluid-absorbent article. Generally, the topsheet is the nearest composition to the wearer of the fluid-absorbent article, hereinafter described as "upper liquid-pervious layer". Contrarily, the term "lower" refers to fluid-absorbent compositions which are away from the wearer of the fluid-absorbent article. Generally, the backsheet is the component which is furthermost away from the wearer of the fluid-absorbent article, hereinafter described as "lower liquid-impervious layer".

As used herein, the term "liquid-pervious" refers to a substrate, layer or a laminate thus permitting liquids, i.e. body fluids such as urine, menses and/or vaginal fluids to readily penetrate through its thickness.

As used herein, the term "liquid-impervious" refers to a substrate, layer or a laminate that does not allow body fluids to pass through in a direction generally perpendicular to the plane of the layer at the point of liquid contact under ordinary use conditions.

As used herein, the term "chassis" refers to fluid-absorbent material comprising the upper liquid-pervious layer and the lower liquid-impervious layer, elastication and closure systems for the absorbent article.

As used herein, the term "hydrophilic" refers to the wettability of fibers by water deposited on these fibers. The term "hydrophilic" is defined by the contact angle and surface tension of the body fluids. According to the definition of Robert F. Gould in the 1964 American Chemical Society publication "Contact angle, wettability and adhesion", a fiber is referred to as hydrophilic, when the contact angle between the liquid and the fiber, especially the fiber sur-face, is less than 90° or when the liquid tends to spread spontaneously on the same surface.

Contrarily, term "hydrophobic" refers to fibers showing a contact angle of greater than 90° or no spontaneously spreading of the liquid across the surface of the fiber.

As used herein, the term "body fluids" refers to any fluid produced and discharged by human or animal body, such as urine, menstrual fluids, faeces, vaginal secretions and the like. As used herein, the term "breathable" refers to a substrate, layer, film or a laminate that allows vapour to escape from the fluid-absorbent article, while still preventing fluids from leakage. Breathable substrates, layers, films or laminates may be porous polymeric films, nonwoven laminates from spunbond and melt- blown layers, laminates from porous polymeric films and nonwovens.

As used herein, the term "longitudinal" refers to a direction running perpendicular from a waist edge to an opposing waist edge of the fluid-absorbent article.

As used herein, the term "fine water-absorbent polymer particles" refers to water-absorbent polymer particles having an average particle diameter of not larger than 300 μm. The fine water-absorbent polymer particles preferably having an average particle diameter of at maximum 150 μm, more preferably at maximum 120 μm.

B. Water-absorbent polymer particles

The water-absorbent polymer particles, e. g. water-absorbent polymer particles H and the water-absorbent polymer particles of the lower layer (92) and the fine water-absorbent polymer particles, are generally prepared by a process, comprising the steps forming water-absorbent polymer particles by polymerizing a monomer solution, comprising

a) at least one ethylenically unsaturated monomer which bears acid groups and may be at least partly neutralized,
b) optionally one or more crosslinker,
c) at least one initiator,
d) optionally one or more ethylenically unsaturated monomers copolymerizable with the monomers mentioned under a),
e) optionally one or more water-soluble polymers, and
f) water,

optionally coating of water-absorbent polymer particles with at least one surface-postcrosslinker and thermal surface-postcrosslinking of the coated water-absorbent polymer particles.

The water-absorbent polymer particles are typically insoluble but swellable in water.

The monomers a) are preferably water-soluble, i.e. the solubility in water at 23° C is typically at least 1 g/100 g of water, preferably at least 5 g/100 g of water, more preferably at least 25 g/100 g of water, most preferably at least 35 g/100 g of water.

Suitable monomers a) are, for example, ethylenically unsaturated carboxylic acids such as acrylic acid, methacrylic acid, maleic acid, and itaconic acid. Particularly preferred monomers are acrylic acid and methacrylic acid. Very particular preference is given to acrylic acid.

Further suitable monomers a) are, for example, ethylenically unsaturated sulfonic acids such as vinylsulfonic acid, styrenesulfonic acid and 2-acrylamido-2-methylpropanesulfonic acid (AMPS).

Impurities may have a strong impact on the polymerization. Preference is given to especially purified monomers a). Useful purification methods are disclosed in WO 2002/055469 A1, WO 2003/078378 A1 and WO 2004/035514 A1. A suitable monomer a) is according to WO 2004/035514 A1 purified acrylic acid having 99.8460% by weight of acrylic acid, 0.0950% by weight of acetic acid, 0.0332% by weight of water, 0.0203% by weight of propionic acid, 0.0001% by weight of furfurals, 0.0001% by weight of maleic anhydride, 0.0003% by weight of diacrylic acid and 0.0050% by weight of hydroquinone monomethyl ether.

Polymerized diacrylic acid is a source for residual monomers due to thermal decomposition. If the temperatures during the process are low, the concentration of diacrylic acid is no more critical and acrylic acids having higher concentrations of diacrylic acid, i.e. 500 to 10,000 ppm, can be used for the inventive process.

The content of acrylic acid and/or salts thereof in the total amount of monomers a) is preferably at least 50 mol%, more preferably at least 90 mol%, most preferably at least 95 mol%.

The acid groups of the monomers a) are typically partly neutralized in the range of 0 to 100 mol%, preferably to an extent of from 25 to 85 mol%, preferentially to an extent of from 50 to 80 mol%, more preferably from 60 to 75 mol%, for which the customary neutralizing agents can be used, preferably alkali metal hydroxides, alkali metal oxides, alkali metal carbonates or alkali metal hydrogen carbonates, and mixtures thereof. Instead of alkali metal salts, it is also possible to use ammonia or organic amines, for example, triethanolamine. It is also possible to use oxides, carbonates, hydrogencarbonates and hydroxides of magnesium, calcium, strontium, zinc or aluminum as powders, slurries or solutions and mixtures of any of the above neutralization agents. Example for a mixture is a solution of sodiumaluminate. Sodium and potassium are particularly preferred as alkali metals, but very particular preference is given to sodium hydroxide, sodium carbonate or sodium hydrogen carbonate, and mixtures thereof. Typically, the neutralization is achieved by mixing in the neutralizing agent as an aqueous solution, as a melt or preferably also as a solid. For example, sodium hydroxide with water content significantly below 50% by weight may be present as a waxy material having a melting point above 23° C. In this case, metered addition as piece material or melt at elevated temperature is possible.

Optionally, it is possible to add to the monomer solution, or to starting materials thereof, one or more chelating agents for masking metal ions, for example iron, for the purpose of stabilization. Suitable chelating agents are, for example, alkali metal citrates, citric acid, alkali metal tartrates, alkali metal lactates and glycolates, pentasodium triphosphate, ethylenediamine tetraacetate, nitrilotriacetic acid, and all chelating agents known under the Trilon® name, for example Trilon® C (pentasodium diethylenetriaminepentaacetate), Trilon® D (trisodium (hydroxyethyl)-ethylenediaminetriacetate), and Trilon® M (methylglycinediacetic acid) and Cublen®.

The monomers a) comprise typically polymerization inhibitors, preferably hydroquinone monoethers, as inhibitor for storage.

The monomer solution comprises preferably up to 250 ppm by weight, more preferably not more than 130 ppm by weight, most preferably not more than 70 ppm by weight, preferably not less than 10 ppm by weight, more preferably not less than 30 ppm by weight and especially about 50 ppm by weight of hydroquinone monoether, based in each case on acrylic acid, with acrylic acid salts being counted as acrylic acid. For example, the monomer solution can be prepared using acrylic acid having appropriate hydroquinone monoether content. The hydroquinone monoethers may, however, also be removed from the monomer solution by absorption, for example on activated carbon.

Preferred hydroquinone monoethers are hydroquinone monomethyl ether (MEHQ) and/or alpha-tocopherol (vitamin E).

Suitable crosslinkers b) are compounds having at least two groups suitable for crosslinking. Such groups are, for example, ethylenically unsaturated groups which can be polymerized by a free-radical mechanism into the polymer chain and functional groups which can form covalent bonds with the acid groups of monomer a). In addition, polyvalent metal ions which can form coordinate bond with at least two acid groups of monomer a) are also suitable crosslinkers b).

The crosslinkers b) are preferably compounds having at least two free-radically polymerizable groups which can be polymerized by a free-radical mechanism into the polymer network. Suitable crosslinkers b) are, for example, ethylene glycol dimethacrylate, diethylene glycol diacrylate, polyethylene glycol diacrylate, allyl methacrylate, trimethylolpropane triacrylate, triallylamine, tetraallylammonium chloride, tetraallyloxyethane, as described in EP 0 530 438 A1, di- and triacrylates, as described in EP 0 547 847 A1, EP 0 559 476 A1, EP 0 632 068 A1, WO 93/21237 A1, WO 2003/104299 A1, WO 2003/104300 A1, WO 2003/104301 A1 and in DE 103 31 450 A1, mixed acrylates which, as well as acrylate groups, comprise further ethylenically unsaturated groups, as described in DE 103 314 56 A1 and DE 103 55 401 A1, or crosslinker mixtures, as described, for example, in DE 195 43 368 A1, DE 196 46 484 A1, WO 90/15830 A1 and WO 2002/32962 A2.

Suitable crosslinkers b) are in particular pentaerythritol triallyl ether, tetraallyloxyethane, polyethyleneglycole dial-lylethers (based on polyethylene glycole having a molecular weight between 400 and 20000 g/mol), N,N'-methylenebisacrylamide, 15-tuply ethoxylated trimethylolpropane, polyethylene glycol diacrylate, trimethylolpro-pane triacrylate and triallylamine.

Very particularly preferred crosslinkers b) are the polyethoxylated and/or -propoxylated glycerols which have been esterified with acrylic acid or methacrylic acid to give di- or triacrylates, as described, for example in WO 2003/104301

A1. Di- and/or triacrylates of 3- to 18-tuply ethoxylated glycerol are particularly advantageous. Very particular preference is given to di- or triacrylates of 1- to 5-tuply ethoxylated and/or propoxylated glycerol. Most preferred are the triacrylates of 3- to 5-tuply ethoxylated and/or propoxylated glycerol and especially the triacrylate of 3-tuply ethoxylated glycerol.

The amount of crosslinker b) is preferably from 0.0001 to 0.6% by weight, more preferably from 0.001 to 0.2% by weight, most preferably from 0.01 to 0.06% by weight, based in each case on monomer a). On increasing the amount of crosslinker b) the centrifuge retention capacity (CRC) decreases and the absorption under a pressure of 21.0 g/cm$^2$ (AUL) passes through a maximum.

The initiators c) used may be all compounds which disintegrate into free radicals under the polymerization conditions, for example peroxides, hydroperoxides, hydrogen peroxide, persulfates, azo compounds and redox initiators. Preference is given to the use of water-soluble initiators. In some cases, it is advantageous to use mixtures of various initiators, for example mixtures of hydrogen peroxide and sodium or potassium peroxodisulfate. Mixtures of hydrogen peroxide and sodium peroxodisulfate can be used in any proportion.

Particularly preferred initiators c) are azo initiators such as 2,2 ' -azobis[2-(2-imidazolin-2-yl)propane] dihydrochloride and 2,2 ' -azobis[2-(5-methyl-2-imidazolin-2-yl)propane] dihydrochloride, 2,2 ' -azobis(2-amidinopropane) dihydrochloride, 4,4 -azobis(4-cyanopentanoic acid), 4,4 ' -azobis(4-cyanopentanoic acid) sodium salt, 2,2 -azobis[2-methyl-N-(2-hydroxyethyl)propionamide], and photoinitiators such as 2-hydroxy-2-methylpropiophenone and 1-[4-(2-hydroxyethoxy)phenyl]-2-hydroxy-2-methyl-1-propan-1-one, redox initiators such as sodium persulfate/hydroxymethylsulfinic acid, ammonium peroxodisulfate/hydroxymethylsulfinic acid, hydrogen peroxide/hydroxymethylsulfinic acid, sodium persulfate/ascorbic acid, ammonium peroxodisulfate/ascorbic acid and hydrogen peroxide/ascorbic acid, photoinitiators such as 1-[4-(2-hydroxyethoxy)phenyl]-2-hydroxy-2-methyl-1-propan-1-one, and mixtures thereof. The reducing component used is, however, preferably a mixture of the sodium salt of 2-hydroxy-2-sulfinatoacetic acid, the disodium salt of 2-hydroxy-2-sulfonatoacetic acid and sodium bisulfite. Such mixtures are obtainable as Brüggolite® FF6 and Brüggolite® FF7 (Brüggemann Chemicals; Heilbronn; Germany). Of course it is also possible within the scope of the present invention to use the purified salts or acids of 2-hydroxy-2-sulfinatoacetic acid and 2-hydroxy-2-sulfonatoacetic acid - the latter being available as sodium salt under the trade name Blancolen® (Brüggemann Chemicals; Heilbronn; Germany).

The initiators are used in customary amounts, for example in amounts of from 0.001 to 5% by weight, preferably from 0.01 to 2% by weight, most preferably from 0.05 to 0.5% by weight, based on the monomers a).

Examples of ethylenically unsaturated monomers d) which are copolymerizable with the monomers a) are acrylamide, methacrylamide, hydroxyethyl acrylate, hydroxyethyl methacrylate, dimethylaminoethyl acrylate, dimethylaminoethyl methacrylate, dimethylaminopropyl acrylate and diethylaminopropyl methacrylate.

Useful water-soluble polymers e) include polyvinyl alcohol, modified polyvinyl alcohol comprising acidic side groups for example Poval® K (Kuraray Europe GmbH; Frankfurt; Germany), polyvinylpyrrolidone, starch, starch derivatives, modified cellulose such as methylcellulose, carboxymethylcellulose or hydroxyethylcellulose, gelatin, polyglycols or polyacrylic acids, polyesters and polyamides, polylactic acid, polyglycolic acid, copolylactic-polyglycolic acid, polyvinylamine, polyallylamine, water soluble copolymers of acrylic acid and maleic acid available as Sokalan® (BASF SE; Ludwigshafen; Germany), preferably starch, starch derivatives and modified cellulose.

For optimal action, the preferred polymerization inhibitors require dissolved oxygen. Therefore, the monomer solution can be freed of dissolved oxygen before the polymerization by inertization, i.e. flowing through with an inert gas, preferably nitrogen. It is also possible to reduce the concentration of dissolved oxygen by adding a reducing agent. The oxygen content of the monomer solution is preferably lowered before the polymerization to less than 1 ppm by weight, more preferably to less than 0.5 ppm by weight.

The water content of the monomer solution is preferably less than 65% by weight, preferentially less than 62% by weight, more preferably less than 60% by weight, most preferably less than 58% by weight.

The monomer solution has, at 20° C, a dynamic viscosity of preferably from 0.002 to 0.02 Pa.s, more preferably from 0.004 to 0.015 Pa.s, most preferably from 0.005 to 0.01 Pa.s.

The monomer solution has, at 20° C, a density of preferably from 1 to 1.3 g/cm$^3$, more preferably from 1.05 to 1.25 g/cm$^3$, most preferably from 1.1 to 1.2 g/cm$^3$.

The monomer solution has, at 20° C, a surface tension of from 0.02 to 0.06 N/m, more preferably from 0.03 to 0.05 N/m, most preferably from 0.035 to 0.045 N/m.

Polymerization

The monomer solution is polymerized. Suitable reactors are, for example, kneading reactors or belt reactors. In the kneader, the polymer gel formed in the polymerization of an aqueous monomer solution or suspension is comminuted continuously by, for example, contrarotatory stirrer shafts, as described in WO 2001/038402 A1. Polymerization on the belt is described, for example, in DE 38 25 366 A1 and US 6,241,928. Polymerization in a belt reactor forms a polymer gel which has to be comminuted in a further process step, for example in an extruder or kneader

To improve the drying properties, the comminuted polymer gel obtained by means of a kneader can additionally

be extruded.

The polymer gel is then preferably dried with a belt dryer until the residual moisture content is

preferably from 0.5 to 15% by weight, more preferably from 1 to 10% by weight, most preferably from 2 to 8% by weight, the residual moisture content being determined by the EDANA recommended test method No. WSP 230.2-05 "Moisture Content". In the case of too high a residual moisture content, the dried polymer gel has too low a glass transition temperature Tg and can be processed further only with difficulty. In the case of too low a residual moisture content, the dried polymer gel is too brittle and, in the subsequent comminution steps, undesirably large amounts of polymer particles with an excessively low particle size are obtained (fines). The solids content of the gel before the drying is preferably from 25 to 90% by weight, more preferably from 35 to 70% by weight, most preferably from 40 to 60% by weight. Optionally, it is, however, also possible to use a fluidized bed dryer or a paddle dryer for the drying operation. Thereafter, the dried polymer gel is ground and classified, and the apparatus used for grinding may typically be single- or multistage roll mills, preferably two- or three-stage roll mills, pin mills, hammer mills or vibratory mills.

Alternatively the water-absorbent polymer particles are produced by polymerizing droplets of the monomer in a surrounding heated gas phase, for example by using a system described in WO 2008/040715 A2, WO 2008/052971 A1, WO 2008/069639 A1 and WO 2008/086976 A1.

The mean particle size or also referred to as average particle diameter of the water-absorbent polymer particles collected as the product fraction, independent of the production process of the water-absorbent polymer particles is preferably above 120 μm, more preferably above 150 μm, most preferably from 250 to 600 μm and very particularly preferable from 300 to 500 μm. The mean particle size may be determined by means of EDANA (European Disposables and Nonwovens Association) recommended test method No. WSP 220.3 (11) "Particle Size Distribution", where the proportions by mass of the screen fractions are plotted in cumulated form and the mean particle size is determined graphically. The mean particle size or average particle diameter here is the value of the mesh size which gives rise to a cumulative 50% by weight.

The fine water-absorbent polymer particles are removed as they are reducing the product performance in superabsorbent products.

According to the present invention the fine water-absorbent polymer particles, which are removed at this process step and/or in later process steps, for example after the surface post-crosslinking or another coating step are collected and agglomerated to result in water-absorbent polymer particles G.

The later process steps, for example the surface post-crosslinking or coating steps are generally described in the following:

Surface-postcrosslinking

Surface-postcrosslinkers are compounds which comprise groups which can form at least two covalent bonds with the carboxylate groups of the polymer particles. Suitable compounds are, for example, polyfunctional amines, polyfunctional amidoamines, polyfunctional epoxides, as described in EP 0 083 022 A2, EP 0 543 303 A1 and EP 0 937 736 A2, di- or polyfunctional alcohols as described in DE 33 14 019 A1, DE 35 23 617 A1 and EP 0 450 922 A2, or $\beta$ -hydroxyalkylamides, as described in DE 102 04 938 A1 and US 6,239,230. Also ethyleneoxide, aziridine, glycidol, oxetane and its derivatives may be used.

Polyvinylamine, polyamidoamines and polyvinylalcohols are examples of multifunctional polymeric surface-postcrosslinkers.

In addition, DE 40 20 780 C1 describes alkylene carbonates, DE 198 07 502 A1 describes 1,3 oxazolidin-2-one and its derivatives such as 2-hydroxyethyl-1,3-oxazolidin-2-one, DE 198 07 992 C1 describes bis- and poly-1,3-oxazolidin-2-ones, EP 0 999 238 A1 describes bis- and poly-1,3-oxazolidines, DE 198 54 573 A1 describes 2 oxotetrahydro-1,3-oxazine and its derivatives, DE 198 54 574 A1 describes N-acyl-1,3 oxazolidin-2-ones, DE 102 04 937 A1 describes cyclic ureas, DE 103 34 584 A1 describes bicyclic amide acetals, EP 1 199 327 A2 de-scribes oxetanes and cyclic ureas, and WO 2003/31482 A1 describes morpholine-2,3-dione and its derivatives, as suitable surface-postcrosslinkers.

In addition, it is also possible to use surface-postcrosslinkers which comprise additional polymerizable ethylenically unsaturated groups e.g .1,3,2-dioxathiolane, as e.g. described in DE 37 13 601 A1.

The at least one surface-postcrosslinker is selected from alkylene carbonates, 1,3 oxazolidin-2-ones, bis- and poly-1,3-oxazolidin-2-ones, bis- and poly-1,3-oxazolidines, 2 oxotetrahydro-1,3-oxazines, N-acyl-1,3 oxazolidin-2-ones, cyclic ureas, bicyclic amide acetals, oxetanes, and morpholine-2,3-diones. Suitable surface-postcrosslinkers are ethylene carbonate, 3-methyl-1,3-oxazolidin-2-one, 3-methyl-3-oxethanmethanol, 1,3-oxazolidin-2-one, 3-(2-hydroxyethyl)-1,3-oxazolidin-2-one, 1,3-dioxan-2-one or a mixture thereof.

It is also possible to use any suitable mixture of surface-postcrosslinkers. It is particularly favorable to use mixtures of 1,3-dioxolan-2-on (ethylene carbonate) and 1,3 oxazolidin-2-ones. Such mixtures are obtainable by mixing and partly reacting of 1,3-dioxolan-2-on (ethylene carbonate) with the corresponding 2-amino-alcohol (e.g. 2-aminoethanol) and may comprise ethylene glycol from the reaction.

It is preferred that at least one alkylene carbonate is used as surface-postcrosslinker. Suitable alkylene carbonates are 1,3-dioxolan-2-on (ethylene carbonate), 4-methyl-1,3-dioxolan-2-on (propylene carbonate), 4,5-dimethyl-1,3-dioxolan-2-on, 4,4-dimethyl-1,3-dioxolan-2-on, 4-ethyl-1,3-dioxolan-2-on, 4-hydroxymethyl-1,3-dioxolan-2-on (glycerine carbonate), 1,3-dioxane-2-on (trimethylene carbonate), 4-methyl-1,3-dioxane-2-on, 4,6-dimethyl-1,3-dioxane-2-on and 1,3-dioxepan-2-on, preferably 1,3-dioxolan-2-on (ethylene carbonate) and 1,3-dioxane-2-on (trimethylene carbonate), most preferably1,3-dioxolan-2-on (ethylene carbonate).

The amount of surface-postcrosslinker is preferably from 0.1 to 10% by weight, more preferably from 0.5 to 7.5% by weight, most preferably from 1 to 5% by weight, based in each case on the polymer.

The content of residual monomers in the water-absorbent polymer particles prior to the coating with the surface-postcrosslinker is in the range from 0.03 to 15% by weight, preferably from 0.05 to 12% by weight, more preferably from 0.1 to 10% by weight, even more preferably from 0.15 to 7.5% by weight, most preferably from 0.2 to 5% by weight, even most preferably from 0.25 to 2.5% by weight.

The moisture content of the water-absorbent polymer particles prior to the thermal surface-postcrosslinking is preferably from 1 to 20% by weight, more preferably from 2 to 15% by weight, most preferably from 3 to 10% by weight.

Polyvalent cations can be applied to the particle surface in addition to the surface-postcrosslinkers before, during or after the thermal surface-postcrosslinking.

The polyvalent cations usable in the process according to the invention are, for example, divalent cations such as the cations of zinc, magnesium, calcium, iron and strontium, trivalent cations such as the cations of aluminum, iron, chromium, rare earths and manganese, tetravalent cations such as the cations of titanium and zirconium, and mixtures thereof. Possible counterions are chloride, bromide, sulfate, hydrogensulfate, methanesulfate, carbonate, hydrogencarbonate, nitrate, hydroxide, phosphate, hydrogenphosphate, dihydrogenphosphate, glycophosphate and carboxylate, such as acetate, glycolate, tartrate, formiate, propionate, 3-hydroxypropionate, lactamide and lactate, and mixtures thereof. Aluminum sulfate, aluminum acetate, and aluminum lactate are preferred. Aluminum lactate is more preferred. Using the inventive process in combination with the use of aluminum lactate, water-absorbent polymer particles having an extremely high total liquid uptake at lower centrifuge retention capacities (CRC) can be prepared.

Apart from metal salts, it is also possible to use polyamines and/or polymeric amines as polyvalent cations. A single metal salt can be used as well as any mixture of the metal salts and/or the polyamines above.

Preferred polyvalent cations and corresponding anions are disclosed in WO 2012/045705 A1 and are expressly incorporated herein by reference. Preferred polyvinylamines are disclosed in WO 2004/024816 A1 and are expressly incorporated herein by reference.

The amount of polyvalent cation used is, for example, from 0.001 to 1.5% by weight, preferably from 0.005 to 1% by weight, more preferably from 0.02 to 0.8% by weight, based in each case on the polymer.

The addition of the polyvalent metal cation can take place prior, after, or cocurrently with the surface-postcrosslinking. Depending on the formulation and operating conditions employed it is possible to obtain a homogeneous surface coating and distribution of the polyvalent cation or an inhomogeneous typically spotty coating. Both types of coatings and any mixes between them are useful within the scope of the present invention.

The surface-postcrosslinking is typically performed in such a way that a solution of the surface-postcrosslinker is sprayed onto the hydrogel or the dry polymer particles. After the spraying, the polymer particles coated with the surface-postcrosslinker are dried thermally and cooled.

The spraying of a solution of the surface-postcrosslinker is preferably performed in mixers with moving mixing tools, such as screw mixers, disk mixers and paddle mixers. Suitable mixers are, for example, vertical Schugi Flexomix® mixers (Hosokawa Micron BV; Doetinchem; the Netherlands), Turbolizers® mixers (Hosokawa Micron BV; Doetinchem; the Netherlands), horizontal Pflugschar® plowshare mixers (Gebr. Lödige Maschinenbau GmbH; Paderborn; Germany), Vrieco-Nauta Continuous Mixers (Hosokawa Micron BV; Doetinchem; the Netherlands), Processall Mixmill Mixers (Processall Incorporated; Cincinnati; US) and Ruberg continuous flow mixers (Gebrüder Ruberg GmbH & Co KG, Nieheim, Germany). Ruberg continuous flow mixers and horizontal Pflugschar® plow-share mixers are preferred. The surface-postcrosslinker solution can also be sprayed into a fluidized bed.

The solution of the surface-postcrosslinker can also be sprayed on the water-absorbent polymer particles during the thermal posttreatment. In such case the surface-postcrosslinker can be added as one portion or in several portions along the axis of thermal posttreatment mixer. In one embodiment it is preferred to add the surface-postcrosslinker at the end of the thermal posttreatment step. As a particular advantage of adding the solution of the surface-postcrosslinker during the thermal posttreatment step it may be possible to eliminate or reduce the technical effort for a separate surface-postcrosslinker addition mixer.

The surface-postcrosslinkers are typically used as an aqueous solution. The addition of nonaqueous solvent

can be used to improve surface wetting and to adjust the penetration depth of the surface-postcrosslinker into the polymer particles.

The thermal surface-postcrosslinking is preferably carried out in contact dryers, more preferably paddle dryers, most preferably disk dryers. Suitable driers are, for example, Hosokawa Bepex® horizontal paddle driers (Hosokawa Micron GmbH; Leingarten; Germany), Hosokawa Bepex® disk driers (Hosokawa Micron GmbH; Leingarten; Germany), Holo-Flite® dryers (Metso Minerals Industries Inc.; Danville; U.S.A.) and Nara paddle driers (NARA Machinery Europe; Frechen; Germany). Moreover, it is also possible to use fluidized bed dryers. In the latter case the reaction times may be shorter compared to other embodiments.

When a horizontal dryer is used then it is often advantageous to set the dryer up with an inclined angle of a few degrees vs. the earth surface in order to impart proper product flow through the dryer. The angle can be fixed or may be adjustable and is typically between 0 to 10 degrees, preferably 1 to 6 degrees, most preferably 2 to 4 degrees.

A contact dryer can be used that has two different heating zones in one apparatus. For example Nara paddle driers are available with just one heated zone or alternatively with two heated zones. The advantage of using a two or more heated zone dryer is that different phases of the thermal post-treatment and/or of the post-surface-crosslinking can be combined.

It is possible to use a contact dryer with a hot first heating zone which is followed by a temperature holding zone in the same dryer. This set up allows a quick rise of the product temperature and evaporation of surplus liquid in the first heating zone, whereas the rest of the dryer is just holding the product temperature stable to complete the reaction.

It is also possible to use a contact dryer with a warm first heating zone which is then followed by a hot heating zone. In the first warm zone the thermal post-treatment is affected or completed whereas the surface-postcrosslinking takes place in the subsequential hot zone.

Typically a paddle heater with just one temperature zone is employed.

A person skilled in the art will depending on the desired finished product properties and the available base polymer qualities from the polymerization step choose any one of these set ups.

The thermal surface-postcrosslinking can be effected in the mixer itself, by heating the jacket, blowing in warm air or steam. Equally suitable is a downstream dryer, for example a shelf dryer, a rotary tube oven or a heatable screw. It is particularly advantageous to mix and dry in a fluidized bed dryer.

Preferred thermal surface-postcrosslinking temperatures are ususally in the range of 100-195° C, mostly in the range of 100 to 180° C, preferably from 120 to 170° C, more preferably from 130 to 165° C, most preferably from 140 to 160° C. The preferred residence time at this temperature in the reaction mixer or dryer is preferably at least 5 minutes, more preferably at least 20 minutes, most preferably at least 40 minutes, and typically at most 120 minutes.

It is preferable to cool the polymer particles after thermal surface-postcrosslinking. The cooling is preferably carried out in contact coolers, more preferably paddle coolers, most preferably disk coolers. Suitable coolers are, for ex-ample, Hosokawa Bepex® horizontal paddle coolers (Hosokawa Micron GmbH; Leingarten; Germany), Hosokawa Bepex® disk coolers (Hosokawa Micron GmbH; Leingarten; Germany), Holo-Flite® coolers (Metso Minerals In-dustries Inc.; Danville; U.S.A.) and Nara paddle coolers (NARA Machinery Europe; Frechen; Germany). Moreover, it is also possible to use fluidized bed coolers.

In the cooler the polymer particles are cooled to temperatures in the range from 20 to 150° C, preferably from 40 to 120° C, more preferably from 60 to 100° C, most preferably from 70 to 90° C. Cooling using warm water is preferred, especially when contact coolers are used.

According to the described production process fine water-absorbent polymer particles are typically removed after post-crosslinking and collected according to the present invention and agglomerated to result in water-absorbent polymer particles G. For agglomeration the fine water-absorbent particles are preferably blended with fine water-absorbent polymer particles collected in at least one different step of the production process.

## Coating

To further improve the properties, the water-absorbent polymer particles can be coated and/or optionally moistened. The internal fluidized bed, the external fluidized bed and/or the external mixer used for the thermal posttreatment and/or a separate coater (mixer) can be used for coating of the water-absorbent polymer particles. Further, the cooler and/or a separate coater (mixer) can be used for coating/moistening of the surface-postcrosslinked water-absorbent polymer particles. Suitable coatings for controlling the acquisition behavior and improving the permeability (SFC or GBP) are, for example, inorganic inert substances, such as water-insoluble metal salts, organic polymers, cationic polymers, anionic polymers and polyvalent metal cations. Suitable coatings for improving the color stability are, for example reducing agents, chelating agents and anti-oxidants. Suitable coatings for dust binding are, for example, polyols. Suitable coatings against the undesired caking tendency of the polymer particles are, for ex-ample, fumed silica, such as Aerosil® 200, and surfactants, such as Span® 20 and Plantacare® 818 UP. Preferred coatings are aluminium dihydroxy monoacetate, aluminium sulfate, aluminium lactate, aluminium 3-hydroxypropionate, zirconium acetate, citric acid or its water soluble

salts, di- and mono-phosphoric acid or their water soluble salts, Blancolen®, Brüggolite® FF7, Cublen®, Span® 20 and Plantacare® 818 UP.

If salts of the above acids are used instead of the free acids then the preferred salts are alkali-metal, earth alkali metal, aluminum, zirconium, titanium, zinc and ammonium salts.

Under the trade name Cublen® (Zschimmer & Schwarz Mohsdorf GmbH & Co KG; Burgstädt; Germany) the following acids and/or their alkali metal salts (preferably Na and K-salts) are available and may be used within the scope of the present invention for example to impart color-stability to the finished product:

1-Hydroxyethane-1,1-diphosphonic acid, Amino-tris(methylene phosphonic acid), Ethylenediamine-tetra(methylene phosphonic acid), Diethylenetriamine-penta(methylene phosphonic acid), Hexamethylene diamine-tetra(methylenephosphonic acid), Hydroxyethylamino-di(methylene phosphonic acid), 2-Phosphonobutane-1,2,4-tricarboxylic acid, Bis(hexamethylenetriamine penta(methylene phosphonic acid).

Most preferably 1-Hydroxyethane-1,1-diphosphonic acid or its salts with sodium, potassium, or ammonium are employed. Any mixture of the above Cublenes® can be used.

Alternatively, any of the chelating agents described before for use in the polymerization can be coated onto the finished product.

Suitable inorganic inert substances are silicates such as montmorillonite, kaolinite and talc, zeolites, activated carbons, polysilicic acids, magnesium carbonate, calcium carbonate, calcium phosphate, aluminum phosphate, barium sulfate, aluminum oxide, titanium dioxide and iron(II) oxide. Preference is given to using polysilicic acids, which are divided between precipitated silicas and fumed silicas according to their mode of preparation. The two variants are commercially available under the names Silica FK, Sipernat®, Wessalon® (precipitated silicas) and Aerosil® (fumed silicas) respectively. The inorganic inert substances may be used as dispersion in an aqueous or water-miscible dispersant or in substance.

When the water-absorbent polymer particles are coated with inorganic inert substances, the amount of inorganic inert substances used, based on the water-absorbent polymer particles, is preferably from 0.05 to 5% by weight, more preferably from 0.1 to 1.5% by weight, most preferably from 0.3 to 1% by weight.

Suitable organic polymers are polyalkyl methacrylates or thermoplastics such as polyvinyl chloride, waxes based on polyethylene, polypropylene, polyamides or polytetrafluoro-ethylene. Other examples are styrene-isoprene-styrene block-copolymers or styrene-butadiene-styrene block-copolymers. Another example are silanole-group bearing polyvinylalcoholes available under the trade name Poval® R (Kuraray Europe GmbH; Frankfurt; Germany).

Suitable cationic polymers are polyalkylenepolyamines, cationic derivatives of polyacrylamides, polyethyleneimines and polyquaternary amines.

Polyquaternary amines are, for example, condensation products of hexamethylenediamine, dimethylamine and epichlorohydrin, condensation products of dimethylamine and epichlorohydrin, copolymers of hydroxyethylcellulose and diallyldimethylammonium chloride, copolymers of acrylamide and $\alpha$ methacryloyloxyethyltrimethylammonium chloride, condensation products of hydroxyethylcellulose, epichlorohydrin and trimethylamine, homopolymers of diallyldimethylammonium chloride and addition products of epichlorohydrin to amidoamines. In addition, polyquaternary amines can be obtained by reacting dimethyl sulfate with polymers such as polyethyleneimines, copolymers of vinylpyrrolidone and dimethylaminoethyl methacrylate or copolymers of ethyl methacrylate and diethylaminoethyl methacrylate. The polyquaternary amines are available within a wide molecular weight range.

However, it is also possible to generate the cationic polymers on the particle surface, either through reagents which can form a network with themselves, such as addition products of epichlorohydrin to polyamidoamines, or through the application of cationic polymers which can react with an added crosslinker, such as polyamines or polyimines in combination with polyepoxides, polyfunctional esters, polyfunctional acids or polyfunctional (meth)acrylates.

It is possible to use all polyfunctional amines having primary or secondary amino groups, such as polyethyleneimine, polyallylamine and polylysine. The liquid sprayed by the process according to the invention preferably comprises at least one polyamine, for example polyvinylamine or a partially hydrolyzed polyvinylformamide.

The cationic polymers may be used as a solution in an aqueous or water-miscible solvent, as dispersion in an aqueous or water-miscible dispersant or in substance.

When the water-absorbent polymer particles are coated with a cationic polymer, the use amount of cationic polymer based on the water-absorbent polymer particles is usually not less than 0.001% by weight, typically not less than 0.01% by weight, preferably from 0.1 to 15% by weight, more preferably from 0.5 to 10% by weight, most preferably from 1 to 5% by weight.

Suitable anionic polymers are polyacrylates (in acidic form or partially neutralized as salt), copolymers of acrylic acid and maleic acid available under the trade name Sokalan® (BASF SE; Ludwigshafen; Germany), and polyvinylalcohols with built in ionic charges available under the trade name Poval® K (Kuraray Europe GmbH; Frankfurt; Germany).

Suitable polyvalent metal cations are $Mg^{2+}$, $Ca^{2+}$, $Al^{3+}$, $Sc^{3+}$, $Ti^{4+}$, $Mn^{2+}$, $Fe^{2+/3+}$, $Co^{2+}$, $Ni^{2+}$, $Cu^{+/2+}$, $Zn^{2+}$, $Y^{3+}$, $Zr^{4+}$, $Ag^{+}$, $La^{3+}$, $Ce^{4+}$, $Hf^{4+}$ and $Au^{+/3+}$; preferred metal cations are $Mg^{2+}$, $Ca^{2+}$, $Al^{3+}$, $Ti^{4+}$, $Zr^{4+}$ and $La^{3+}$; particularly preferred metal cations are $Al^{3+}$, $Ti^{4+}$ and $Zr^{4+}$. The metal cations may be used either alone or in a mixture with one

another. Suitable metal salts of the metal cations mentioned are all of those which have a sufficient solubility in the solvent to be used. Particularly suitable metal salts have weakly complexing anions, such as chloride, hydroxide, carbonate, acetate, formiate, propionate, nitrate, sulfate and methanesulfate. The metal salts are preferably used as a solution or as a stable aqueous colloidal dispersion. The solvents used for the metal salts may be water, alcohols, ethylenecarbonate, propylenecarbonate, dimethylformamide, dimethyl sulfoxide and mixtures thereof. Particularly preference is given to water and water/alcohol mixtures, such as water/methanol, water/isopropanol, water/1,3-propanediole, water/1,2-propandiole/1,4-butanediole or water/propylene glycol.

When the water-absorbent polymer particles are coated with a polyvalent metal cation, the amount of polyvalent metal cation used, based on the water-absorbent polymer particles, is preferably from 0.05 to 5% by weight, more preferably from 0.1 to 1.5% by weight, most preferably from 0.3 to 1% by weight.

Suitable reducing agents are, for example, sodium sulfite, sodium hydrogensulfite (sodium bisulfite), sodium dithionite, sulfinic acids and salts thereof, ascorbic acid, sodium hypophosphite, sodium phosphite, and phosphinic acids and salts thereof. Preference is given, however, to salts of hypophosphorous acid, for example sodium hypophosphite, salts of sulfinic acids, for example the disodium salt of 2-hydroxy-2-sulfinatoacetic acid, and addition products of aldehydes, for example the disodium salt of 2-hydroxy-2 sulfonatoacetic acid. The reducing agent used can be, however, a mixture of the sodium salt of 2-hydroxy-2-sulfinatoacetic acid, the disodium salt of 2-hydroxy-2 sulfonatoacetic acid and sodium bisulfite. Such mixtures are obtainable as Brüggolite® FF6 and Brüggolite® FF7 (Brüggemann Chemicals; Heilbronn; Germany). Also useful is the purified 2-hydroxy-2 sulfonatoacetic acid and its sodium salts, available under the trade name Blancolen® from the same company.

The reducing agents are typically used in the form of a solution in a suitable solvent, preferably water. The reducing agent may be used as a pure substance or any mixture of the above reducing agents may be used.

When the water-absorbent polymer particles are coated with a reducing agent, the amount of reducing agent used, based on the water-absorbent polymer particles, is preferably from 0.01 to 5% by weight, more preferably from 0.05 to 2% by weight, most preferably from 0.1 to 1% by weight.

Suitable polyols are polyethylene glycols having a molecular weight of from 400 to 20000 g/mol, polyglycerol, 3- to 100-tuply ethoxylated polyols, such as trimethylolpropane, glycerol, sorbitol, mannitol, inositol, pentaerythritol and neopentyl glycol. Particularly suitable polyols are 7- to 20-tuply ethoxylated glycerol or trimethylolpropane, for example Polyol TP 70® (Perstorp AB, Perstorp, Sweden). The latter have the advantage in particular that they lower the surface tension of an aqueous extract of the water-absorbent polymer particles only insignificantly. The polyols are preferably used as a solution in aqueous or water-miscible solvents.

The polyol can be added before, during, or after surface-crosslinking. Preferably it is added after surface-crosslinking. Any mixture of the above listed polyols may be used.

When the water-absorbent polymer particles are coated with a polyol, the use amount of polyol, based on the water-absorbent polymer particles, is preferably from 0.005 to 2% by weight, more preferably from 0.01 to 1% by weight, most preferably from 0.05 to 0.5% by weight.

The coating is preferably performed in mixers with moving mixing tools, such as screw mixers, disk mixers, paddle mixers and drum coater. Suitable mixers are, for example, horizontal Pflugschar® plowshare mixers (Gebr. Lödige Maschinenbau GmbH; Paderborn; Germany), Vrieco-Nauta Continuous Mixers (Hosokawa Micron BV; Doetinchem; the Netherlands), Processall Mixmill Mixers (Processall Incorporated; Cincinnati; US) and Ruberg continuous flow mixers (Gebrüder Ruberg GmbH & Co KG, Nieheim, Germany). Moreover, it is also possible to use a fluidized bed for mixing.

According to the described production process fine water-absorbent polymer particles are typically removed also after coating and collected according to the present invention and agglomerated to result in water-absorbent polymer particles G. For agglomeration the fine water-absorbent polymer particles are preferably blended with fine water-absorbent polymer particles collected in at least one different step of the production process.

Agglomeration

According to the production process fine water-absorbent polymer particles are typically removed after polymerization and/or post-crosslinking and/or coating.

According to the invention these fine water-absorbent polymer particles are used for producing water-absorbent polymer particles G.

Therefore, these fine water-absorbent polymer particles are collected. The fine water-absorbent polymer particles which are agglomerated are non-surface crosslinked and/or surface-crosslinked and/or coated or preferably be a mixture of at least two of them.

The water-absorbent polymer particles G are obtainable by agglomerating non-surface post-crosslinked fine water-absorbent polymer particles, surface post-crosslinked fine water-absorbent polymer particles or preferably by agglomerating a blend of non-surface post-crosslinked fine water-absorbent polymer particles and surface post-crosslinked fine water-absorbent polymer particles drying, grinding, sieving and classification of the agglomerated water-absorbent polymer particles. Alternatively by agglomerating a blend of non-surface post-crosslinked fine water-absorbent polymer particles and/or surface post-crosslinked fine water-absorbent polymer and/or coated fine water-absorbent polymer particles, drying, grinding, sieving and classification of the agglomerated water-absorbent polymer particles.

It is preferred, that this mixture or blend comprises at least 50 % by weight of non-surface post-crosslinked fine water-absorbent polymer particles and at maximum 50 % by weight of surface post-crosslinked fine water-absorbent polymer particles based on the sum of fine water-absorbent polymer particles.

More preferably at least 60 % by weight of non-surface post-crosslinked fine water-absorbent polymer particles and at maximum 40 % by weight of surface post-crosslinked fine water-absorbent polymer particles. Most preferably at least 66 % by weight of non-surface post-crosslinked fine water-absorbent polymer particles and at maximum 34 % by weight of surface post-crosslinked fine water-absorbent polymer particles. It is furthermore preferred that these mixture comprises at least 70% by weight of non-surface post-crosslinked fine water-absorbent polymer particles and at maximum 30% by weight of surface post-crosslinked fine water-absorbent polymer particles, preferably the mixture comprises at least 75 % by weight of non-surface post-crosslinked fine water-absorbent polymer particles and at maximum 25 % by weight of surface post-crosslinked fine water-absorbent polymer particles.

According to an embodiment of the invention the ratio of the non-surface post-crosslinked fine water-absorbent polymer particles to the surface post-crosslinked fine water-absorbent polymer particles is at least 2 to 1 by weight.

According to another embodiment of the invention the ratio of the non-surface post-crosslinked fine water-absorbent polymer particles to the surface post-crosslinked fine water-absorbent polymer particles is at least 3 to 1 by weight.

According to the invention the fine water-absorbing polymer particles are agglomerated to obtain water-absorbent polymer particles G by using a solution or suspension comprising,

a) 0,04 to 1,2 % by weight water-soluble or water-dispersible polymeric binders or a mixture thereof, based on the water-absorbent polymer particles,
b) 20 to 70% by weight of water based on the water-absorbent polymer particles, and
c) 5 to 20% by weight of a water-miscible organic solvent based on the water-absorbent polymer particles.

For agglomeration the solution or suspension is sprayed onto the fine water-absorbing polymeric particles. The spraying with the solution can, for example, be carried out in mixers having moving mixing implements, such as screw mixers, paddle mixers, disk mixers, plowshare mixers and shovel mixers. Useful mixers include for example Lödige® mixers, Bepex® mixers, Nauta® mixers, Processall® mixers and Schugi® mixers. Vertical mixers are preferred. Fluidized bed apparatuses are particularly preferred.

The performance of the agglomeration is known to those skilled in the art and is not subject to any restrictions.

The solution or suspension preferably comprises water and water-miscible organic solvents, such as alcohols, tetrahydrofuran and acetone; water-soluble and/or water-dispersible polymeric binders are used in addition.

Water-soluble polymeric binders include, but are not limited to: carboxymethyl cellulose, starch, dextran, polyvinylamine, polyethyleneimine, polyvinylalcohol, polyacrylic acid and its salts, polyethylene oxide, polyethyleneglycol and chitosan.

Water-dispersible polymeric binders according to the invention may include, but are not limited to: homo- and copolymers of vinyl esters, in particular vinyl acetate homopolymers and vinyl acetate copolymers with ethylene, acrylates, maleic acid esters, vinyl-amides, other vinylacyl derivatives and/or homo- and co-polymers of acrylic and methacrylic acid esters, such as copolymers of methyl methacrylate, n-butyl acrylate, or 2-ethylhexyl acrylate.

Copolymers based on vinyl esters, acrylic acid esters, and methacrylic acid esters comprise comonomers, for example, styrene, butadiene, a vinylamide, an olefinically unsaturated carboxylic acid or derivatives thereof, a vinylphosphonic acid or derivative thereof, or a (poly)glycol ester of unsaturated acids. Examples of vinylamides include, but are not limited to, N-vinylformamide, N-vinyl-N-methylacetamide, and N-vinylpyrrolidone.

Examples of olefinically unsaturated carboxylic acids are, for example, acrylic acid, methacrylic acid, itaconic acid, and maleic acid. Examples of derivatives of these olefinically unsaturated carboxylic acids are, for example, amides, such as (meth)acrylamide, N-tert-butyl(meth)acrylamide, and N-isopropyl (meth)acryl-amide, and the N-methylolamides or ethers of N-methylolamides, hemiamides, and imides of aliphatic amines, as well as acrylonitrile. Examples of derivatives of vinylphosphonic acid are, for example, the mono- and diesters of C1-C18 alcohols, for example, the methyl, propyl, or stearyl esters.

Glycol esters of unsaturated acids include hydroxyethyl (meth)acrylate or esters of acrylic and methacrylic acid with polyalkylene oxide compounds of the general formula

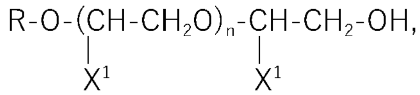

wherein $X^1$ is hydrogen or methyl, n is 0 to 50, and R is an alkyl, alkaryl, or cycloalkyl $C_1$-$C_{24}$ radical, for example, octylphenyl, dodecyl, or nonylphenyl.

Other suitable water-dispersible polymeric binders are polyacetals, i.e., reaction products of polyvinyl alcohols with aldehydes, such as, for example, butyraldehyde; polyurethane polymers prepared from polyhydric alcohols and isocyanates, for example, prepared from polyester and/or polyether diols and, for example, toluene-2,4- or 2,6-diisocyanate, methylene-4,4-di(phenyl isocyanate), or hexamethylene diisocyanate; polyureas, i.e., polymers prepared from diamines and diisocyanates or by polycondensation of diamines with carbon dioxide, phosgene, carboxylic acid esters (for example, activated diphenyl carbonates), or urea, or by reaction of diisocyanates with water; a polysiloxane, i.e., linear dimethylpolysiloxane having end groups blocked in different ways; polyamides and copolyamides; polyesters, i.e., polymers prepared by ring-opening polymerization of lactones or by condensation of hydroxycarboxylic acids or diols and dicarboxylic acid derivatives; epoxy resins prepared from polyepoxides by addition reactions with suitable curing agents or by polymerization by way of epoxide groups; polycarbonates prepared by reaction of diglycols or bisphenols with phosgene or carbonic acid diesters in condensation or transesterification reactions; and mixtures thereof.

Preferred water-dispersible polymeric binders are homo- and co-polymers of acrylic acid esters and methacrylic acid esters, and polymers based on polyacetals. Mixtures of two or more polymers also can be used. The mixture ratios are noncritical and are judiciously determined by persons skilled in the art to fit the particular circumstances.

According to the invention a dispersion containing water-dispersible polymeric binders comprising about 5% to about 75%, by weight, of the polymer in water and/or suitable water-miscible organic solvents. The polymer is dispersed in a sufficient amount of water and/or suitable water-miscible organic solvents to allow the polymer to be readily and homogeneously applied to the surfaces of the fine water-absorbent polymer particles. The suitable water-miscible organic solvents for the polymer can be, but is not limited to, an alcohol, or a glycol, such as methanol, ethanol, ethylene glycol, or propylene glycol, and mixtures thereof. Often, the water-dispersible polymeric binders are applied as an emulsion containing the polymer, water, optional organic solvents, emulsifiers, and other ingredients typically used in the preparation of emulsions.

Further suitable organic solvents include, but are not limited to, aliphatic and aromatic hydrocarbons, alcohols, ethers, esters, and ketones, for example, n-hexane, cyclohexane, toluene, xylene, methanol, ethanol, i-propanol, ethylene glycol, 1,2-propanediol, glycerol, diethyl ether, methyltriglycol, a polyethylene glycol having an average molecular weight (Mw) of 200-10,000, ethyl acetate, n-butyl acetate, acetone, 2-butanone, and mixtures thereof.

According to the invention it is preferred that, 0,04 to 0,8% by weight of water-soluble or water-dispersible polymeric binders or a mixture thereof, preferably polyacrylates are used for agglomeration, more preferably from 0.04 to 0.42% by weight, most preferably from 0.16 to 0.42% by weight based on the sum of fine water-absorbent polymer particles.

It is further preferred that 20 to 70 % by weight of water are used, more preferably 30 to 60 % by weight based on the sum of fine water-absorbent polymer particles.

Furthermore preferably 5 to 20% by weight of an organic solvent are present, more preferably from 5 to 15% by weight, most preferably from 5 to 10% by weight based on the sum of fine water-absorbent polymer particles.

The features of the resulting agglomerated water-absorbent polymer particles G are controlled or influenced by the type and amount of binder or the amount of water used in the agglomeration process. An increase of the amount of water e.g. results in a higher amount of resulting particles/agglomerates with a size above 150 μm.

Furthermore, the resulting agglomerated water-absorbent polymer particles G can be e.g. surface-post-crosslinked and/or coated to further adjust their properties.

The average particle diameter of the water-absorbent particles G is preferably from 200 to 550 μm, more preferably from 250 to 500 μm, most preferably from 350 to 450 μm.

According to the invention the resulting water-absorbent polymer particles G having a bulk density of 0.55 g/ml or lower.

Preferably the water-absorbent polymer particles G having a vortex of less than 25s.

According to the invention the vortex of water-absorbent polymer particles G is preferably lower than the vortex of the non-agglomerated non-surface post-crosslinked fine water-absorbent polymer particles, surface post-crosslinked fine water-absorbent polymer particles or mixtures thereof used for its production.

Preferably the water-absorbent polymer particles G according to the invention produced by agglomeration of fine water-absorbent polymer particles having a vortex of less than 25 s and a bulk density of 0.55 g/ml or lower.

The water-absorbent polymer particles G having an absorption under load of 0.3 psi (AAP 0.3 psi or AUL 0.3 psi) of at least 10 g/g, preferably of at least 15 g/g, more preferably of at least 18 g/g, most preferably of at least 20 g/g.

The bulk density of the water-absorbent particles G is preferably less than 0.55 g/ml, more preferably less than 0.50 g/ml, preferentially less than 0.49 g/ml, most preferably less than 0.47 g/ml.

Preferably the bulk density of the water-absorbent particles G is between 0.41 g/ml and 0.49 g/ml.

Water-absorbent polymer particles G having a centrifuge retention capacity (CRC) of at least 15 g/g, more preferably of at least 18 g/g, most preferably of at least 20 g/g.

Preferred water-absorbent polymer particles G according to the invention produced by agglomeration of fine water-absorbent polymer particles having a vortex of less than 15 s and a bulk density of 0.49 g/ml or lower, a CRC of at least 15 g/g and an AUL 0.3 psi of at least 10 g/g.

More preferred water-absorbent polymer particles G according to the invention produced by agglomeration of fine water-absorbent polymer particles having a vortex of less than 25 s and a bulk density of 0.55 g/ml or lower, a CRC of at least 15 g/g and an AUL 0.3 psi of at least 15 g/g

C. Fluid-absorbent articles

The fluid-absorbent article comprises of

(A) an upper liquid-pervious layer (89)
(B) a lower liquid-impervious layer (83)
(C) a fluid-absorbent core (80) between (89) and (83) comprising

at least two layers, wherein each layer comprising from 0 to 10% by weight a fibrous material and from 90 to 100% by weight water-absorbent polymer particles; preferably from 0 to 5% by weight a fibrous material and from 95 to 100% by weight water-absorbent polymer particles;
more preferably from 0% by weight a fibrous material and 100 by weight water-absorbent polymer particles;
based on the sum of water-absorbent polymer material and fibrous material,

(D) an acquisition-distribution layer (70) between (A) and (C) and
(F) other optional components.

Preferably the fluid-absorbent core (80) between (89) and (83) comprising
an upper tissue layer (95), an upper layer comprising water-absorbent polymer particles (91) and a lower layer comprising water-absorbent polymer particles (92), at least one layer of nonwoven material (94) sandwiched between the upper layer (91) and lower layer (92) com-prising water absorbent polymer particles.

Fluid-absorbent articles are understood to mean, for example, incontinence pads and incontinence briefs for adults or diapers and training pants for babies. Suitable fluid-absorbent articles including fluid-absorbent compositions comprising fibrous materials and optionally water-absorbent polymer particles to form fibrous webs or matrices for the substrates, layers, sheets and/or the fluid-absorbent core.

Suitable fluid-absorbent articles are composed of several layers whose individual elements must show preferably definite functional parameter such as dryness for the upper liquid-pervious layer (89), vapor permeability without wetting through for the lower liquid-impervious layer (83), a flexible, vapor permeable and thin fluid-absorbent core (80), showing fast absorption rates and being able to retain highest quantities of body fluids, and an acquisition-distribution layer (70) between the upper layer (89) and the core (80), acting as transport and distribution layer of the discharged body fluids. These individual elements are combined such that the resultant fluid-absorbent article meets overall criteria such as flexibility, water vapor breathability, dryness, wearing comfort and protection on the user facing side, and concerning liquid retention, rewet and prevention of wet through on the garment side. The specific combination of these layers provides a fluid-absorbent article delivering both high protection levels as well as high comfort to the consumer.

For fluid-absorbent articles it is advantageous especially in respect to fluid distribution to have acquisition-distribution layers. For fluid-absorbent articles that possess a fluid- absorbent core comprising very permeable water-absorbent polymer particles a small and thin acquisition-distribution layer (70) can be used.

The acquisition-distribution layer (70) acts as transport and distribution layer of the discharged body fluids and is typically optimized to affect efficient liquid distribution with the underlying fluid-absorbent core. Hence, for quick temporary liquid retention it provides the necessary void space while its area coverage of the underlying fluid-absorbent core must affect the necessary liquid distribution and is adopted to the ability of the fluid-absorbent core to quickly dewater the acquisition-distribution layer.

Methods to make fluid absorbent articles are for example described in the following publications and literature cited therein and are expressly incorporated into the present invention: EP 2 301 499 A1, EP 2 314 264 A1, EP 2 387 981 A1, EP 2 486 901 A1, EP 2 524 679 A1, EP 2 524 679 A1, EP 2 524 680 A1, EP 2 565 031 A1, US 6,972,011, US 2011/0162989, US2011/0270204, WO 2010/004894 A1, WO 2010/004895 A1, WO 2010/076857 A1, WO2010/082373

A1, WO 2010/118409 A1, WO 2010/133529 A2, WO 2010/143635 A1, WO2011/084981 A1, WO 2011/086841 A1, WO 2011/086842 A1, WO 2011/086843 A1, WO2011/086844 A1, WO 2011/117997 A1, WO 2011/136087 A1, WO 2012/048879 A1, WO2012/052173 A1 und WO 2012/052172 A1.

Figure 1 is a schematic view of a fluid absorbent article

The fluid-absorbent article comprises an absorbent core (80) comprising at least two layers of water-absorbent polymer particles, top (91), bottom (92) optionally sandwiched by at least two tissue layers, top (95) and bottom (96) and comprising at least one nonwoven (94) (e.g. high loft air thru bond nonwoven layer) sandwiched by the at least two layers of water-absorbent polymer particles (91, 92). The layers may be connected to each other, e. g. by adhesive, ultrasonic bonding or any other suitable method. The total core structure (80) is optionally surrounded/wrapped by a further nonwoven sheet or tissue layer (86), the so called core wrap, also optionally connected by an adhesive to the sandwich structured absorbent core (80).

Furthermore the absorbent article comprises an acquisition distribution layer (70) on top of the core (80) or core wrap (86) respectively below the upper liquid-pervious sheet (89) (e. g. embossed spunbond nonwoven), and a lower liquid-impervious sheet (83).

Leg cuffs (81) and some elastics (88) may be also present.

Liquid-pervious sheet or liquid pervious layer (89)

The liquid-pervious sheet (89) is the layer which is in direct contact with the skin. Thus, the liquid-pervious sheet (89) is preferably compliant, soft feeling and non-irritating to the consumer ’ s skin. Generally, the term "liquid-pervious" is understood thus permitting liquids, i.e. body fluids such as urine, menses and/or vaginal fluids to readily penetrate through its thickness. The principle function of the liquid-pervious sheet (89) is the acquisition and transport of body fluids from the wearer towards the fluid-absorbent core. Typically liquid pervious layers (89) are formed from any materials known in the art such as nonwoven material, films or combinations thereof. Suitable liquid-pervious sheets (89) consist of customary synthetic or semisynthetic fibers or bi-component fibers or films of polyester, polyolefins, rayon or natural fibers or any combinations thereof. In the case of nonwoven materials, the fibers should generally be bound by binders such as polyacrylates. Additionally the liquid-pervious sheet may contain elastic compositions thus showing elastic characteristics allowing to be stretched in one or two directions.

Suitable synthetic fibers are made from polyvinyl chloride, polyvinyl fluoride, polytetrafluorethylene, polyvinylidene chloride, polyacrylics, polyvinyl acetate, polyethylvinyl acetate, non-soluble or soluble polyvinyl alcohol, polyolefins such as polyethylene, polypropylene, polyamides, poly-esters, polyurethanes, polystyrenes and the like

Examples for films are apertured formed thermoplastic films, apertured plastic films, hydroformed thermoplastic films, reticulated thermoplastic films, porous foams, reticulated foams,
and thermoplastic scrims.

Examples of suitable modified or unmodified natural fibers include cotton, bagasse, kemp, flax, silk, wool, wood pulp, chemically modified wood pulp, jute, rayon, ethyl cellulose, and cellulose acetate.

The fibrous material may comprise only natural fibers or synthetic fibers or any combination thereof. Preferred materials are polyester, rayon and blends thereof, polyethylene, and polypropylene. The fibrous material as a component of the fluid-absorbent compositions may be hydrophilic, hydrophobic or can be a combination of both hydrophilic and hydrophobic fibers. The selection of the ratio hydrophilic/hydrophobic and accordingly the amount of hydrophilic and hydrophobic fibers within fluid-absorbent composition will depend upon fluid han-dling properties and the amount of water-absorbent polymer particles of the resulting fluid-absorbent composition.

Examples for hydrophilic fibers are cellulosic fibers, modified cellulosic fibers, rayon, polyes- ter fibers such as polyethylen terephthalate, hydrophilic nylon and the like. Hydrophilic fi- bers can also be obtained from hydrophobic fibers which are hydrophilized by e. g. surfactant-treating or silica-treating. Thus, hydrophilic thermoplastic fibers derived from polyolefins such as polypro-pylene, polyamides, polystyrenes or the like by surfactant-treating or silica-treating.

To increase the strength and the integrity of the upper-layer, the fibers should generally show bonding sites, which act as crosslinks between the fibers within the layer. Technologies for consolidating fibers in a web are mechanical bonding, thermal bonding and chemical bonding. In the process of mechanical bonding the fibers are entangled mechanically, e.g., by water jets (spunlace) to give integrity to the web. Thermal bonding is car-ried out by means of raising the temperature in the presence of low-melting polymers. Examples for thermal bonding processes are spunbonding, through-air bonding and resin bonding.

Preferred means of increasing the integrity are thermal bonding, spunbonding, resin bonding, through-air bonding and/or spunlace.

In the case of thermal bonding, thermoplastic material is added to the fibers. Upon thermal treatment at least a portion of this thermoplastic material is melting and migrates to intersec-tions of the fibers caused by capillary effects. These intersections solidify to bond sites after cooling and increase the integrity of the fibrous matrix. Moreover, in the case of chemically stiffened cellulosic fibers, melting and migration of the thermoplastic material has the effect of increasing the pore size of the resultant fibrous layer while maintaining its density and basis weight. Upon wetting, the structure and integrity of the layer remains stable. In summary, the addition of thermoplastic material leads to improved

fluid permeability of discharged body fluids and thus to improved acquisition properties.

Suitable thermoplastic materials including polyolefins such as polyethylene and polypropylene, polyesters, copolyesters, polyvinyl acetate, polyethylvinyl acetate, polyvinyl chloride, polyvinylidene chloride, polyacrylics, polyamides, copolyamides, polystyrenes, polyurethanes and copolymers of any of the mentioned polymers.

Suitable thermoplastic fibers can be made from a single polymer that is a monocomponent fiber. Alternatively, they can be made from more than one polymer, e.g., bi-component or multi-component fibers. The term "bicomponent fibers" refers to thermoplastic fibers that comprise a core fiber made from a different fiber material than the shell. Typically, both fiber materials have different melting points, wherein generally the sheath melts at lower temper- atures. Bi-component fibers can be concentric or eccentric depending whether the sheath has a thickness that is even or uneven through the cross-sectional area of the bicomponent fiber. Advantage is given for eccentric bi-component fibers showing a higher compressive strength at lower fiber thickness. Further bi-component fibers can show the feature "uncrimped" (unbent) or "crimped" (bent), further bi-component fibers can demonstrate differing aspects of surface lubricity.

Examples of bi-component fibers include the following polymer combinations: polyethylene/polypropylene, polyethylvinyl acetate/polypropylene, polyethylene/polyester, polypropylene/polyester, copolyester/polyester and the like.

Suitable thermoplastic materials have a melting point of lower temperatures that will damage the fibers of the layer; but not lower than temperatures, where usually the fluid-absorbent articles are stored. Preferably the melting point is between about 75° C and 175° C.

The typical length of thermoplastic fibers is from about 0.4 to 6 cm, preferably from about 0.5 to 1 cm. The diameter of thermoplastic fibers is defined in terms of either denier (grams per 9000 meters) or dtex (grams per 10 000 meters). Typical thermoplastic fibers have a dtex in the range from about 1.2 to 20, preferably from about 1.4 to 10.

A further mean of increasing the integrity of the fluid-absorbent composition is the spunbond-ing technology. The nature of the production of fibrous layers by means of spunbonding is based on the direct spinning of polymeric granulates into continuous filaments and subsequently manufacturing the fibrous layer.

Spunbond fabrics are produced by depositing extruded, spun fibers onto a moving belt in a uni-form random manner followed by thermal bonding the fibers. The fibers are separated during the web laying process by air jets. Fiber bonds are generated by applying heated rolls or hot needles to partially melt the polymer and fuse the fibers together. Since molecu- lar orientation increases the melting point, fibers that are not highly drawn can be used as thermal binding fibers. Polyethylene or random ethylene/propylene copolymers are used as low melting bonding sites.

Besides spunbonding, the technology of resin bonding also belongs to thermal bonding subjects. Using this technology to generate bonding sites, specific adhesives, based on e.g. epoxy, polyurethane and acrylic are added to the fibrous material and the resulting matrix is thermically treated. Thus the web is bonded with resin and/or thermal plastic resins dispersed within the fibrous material.

As a further thermal bonding technology through-air bonding involves the application of hot air to the surface of the fibrous fabric. The hot air is circulated just above the fibrous fabric, but does not push through the fibrous fabric. Bonding sites are generated by the addition of binders. Suitable binders used in through-air thermal bonding include crystalline binder fibers, bi-component binder fibers, and powders. When using crystalline binder fibers or powders, the binder melts entirely and forms molten droplets throughout the nonwoven's cross-section. Bonding occurs at these points upon cooling. In the case of sheath/core binder fibers, the sheath is the binder and the core is the carrier fiber. Products manufactured using through-air ovens tend to be bulky, open, soft, strong, extensible, breathable and absorbent. Through-air bonding followed by immediate cold calendering results in a thick-ness between a hot roll calendered product and one that has been though-air bonded without compression. Even after cold calendering, this product is softer, more flexible and more extensible than area-bond hot-calendered material.

Spunlacing ("hydroentanglement") is a further method of increasing the integrity of a web. The formed web of loose fibers (usually air-laid or wet-laid) is first compacted and prewetted to eliminate air pockets. The technology of spunlacing uses multiple rows of fine high-speed jets of water to strike the web on a porous belt or moving perforated or patterned screen so that the fibers knot about one another. The water pressure generally increases from the first to the last injectors. Pressures as high as 150 bar are used to direct the water jets onto the web. This pressure is sufficient for most of the nonwoven fibers, although higher pressures are used in specialized applications.

The spunlace process is a nonwovens manufacturing system that employs jets of water to entangle fibers and thereby provide fabric integrity. Softness, drape, conformability, and relatively high strength are the major characteristics of spunlace nonwoven.

In newest researches benefits are found in some structural features of the resulting liquid-pervious layers. For example, the thickness of the layer is very important and influences together with its x-y dimension the acquisition-distribution behaviour of the layer. If there is further some profiled structure integrated, the acquisition-distribution behaviour can be directed depending on the three-dimensional structure of the layer. Thus 3D-polyethylene in the function of liquid-pervious layer is preferred.

Thus, suitable liquid-pervious sheets (89) are nonwoven layers formed from the fibers above by thermal bonding,

spunbonding, resin bonding or through-air bonding. Further suitable liquid-pervious layers are 3D-polyethylene layers and spunlace.

Preferably the 3D-polyethylene layers and spunlace show basis weights from 12 to 22 gsm.

Typically liquid-pervious sheets (89) extend partially or wholly across the fluid-absorbent structure and can extend into and/or form part of all the preferred sideflaps, side wrapping elements, wings and ears.

Liquid-impervious sheet or liquid impervious layer (83)

The liquid-impervious sheet (83) prevents the exudates absorbed and retained by the fluid-absorbent core from wetting articles which are in contact with the fluid-absorbent article, as for example bedsheets, pants, pyjamas and undergarments. The liquid-impervious sheet (83) may thus comprise a woven or a nonwoven material, polymeric films such as thermoplastic film of polyethylene or polypropylene, or composite materials such as film-coated nonwoven material.

Suitable liquid-impervious sheets (83) include nonwoven, plastics and/or laminates of plastic and nonwoven. Both, the plastics and/or laminates of plastic and nonwoven may appropriately be breathable, that is, the liquid-impervious layer (83) can permit vapors to escape from the fluid-absorbent material. Thus the liquid-impervious sheet has to have a definite water vapor transmission rate and at the same time the level of impermeability. To combine these features, suitable liquid-impervious layers including at least two layers, e.g. laminates from fibrous nonwoven having a specified basis weight and pore size, and a continuous three-dimensional film of e.g. polyvinylalcohol as the second layer having a specified thickness and optionally having pore structure. Such laminates acting as a barrier and showing no liquid transport or wet through. Thus, suitable liquid-impervious layers comprising at least a first breathable layer of a porous web which is a fibrous nonwoven, e.g. a composite web of a meltblown nonwoven layer or of a spunbonded nonwoven layer made from synthetic fibers and at least a second layer of a resilient three dimensional web consisting of a liquid-impervious polymeric film, e.g. plastics optionally having pores acting as capillaries, which are preferably not perpendicular to the plane of the film but are disposed at an angle of less than 90° relative to the plane of the film.

Suitable liquid-impervious sheets are permeable for vapor. Preferably the liquid-impervious sheet is constructed from vapor permeable material showing a water vapor transmission rate (WVTR) of at least about 100 gsm per 24 hours, preferably at least about 250 gsm per 24 hours and most preferred at least about 500 gsm per 24 hours.

Preferably the liquid-impervious sheet (83) is made of nonwoven comprising hydrophobic materials, e.g. synthetic fibers or a liquid-impervious polymeric film comprising plastics e.g. polyethylene. The thickness of the liquid-impervious sheet is preferably 15 to 30 $\mu$m.

Further, the liquid-impervious sheet (83) is preferably made of a laminate of nonwoven and plastics comprising a nonwoven having a density of 12 to 15 gsm and a polyethylene layer having a thickness of about 10 to 20 $\mu$m.

The typically liquid-impervious sheet (83) extends partially or wholly across the fluid- absorbent structure and can extend into and/or form part of all the preferred sideflaps, side wrapping elements, wings and ears.

Fluid-absorbent Core (80)

The fluid-absorbent core (80) is disposed between the upper liquid-pervious sheet(89) and the lower liquid-impervious sheet (83). According to the present invention the fluid-absorbent core (80) can be formed from absorbent paper.

In order to increase the integrity of the fluid-absorbent core (80), the core may optionally provid-ed with a cover (86) (e.g. tissue wrap). This cover (86) may be at the top and/or at the bottom of the fluid-absorbent core (80) with bonding at lateral juncture and/or bonding at the distal juncture by hot-melt, ultrasonic bonding, thermal bonding or combination of bonding techniques know to persons skilled in the art. Further, this cover (86) may include the whole fluid-absorbent core with a unitary sheet of material and thus function as a wrap. Wrapping is possible as a full wrap, a partial wrap or as a C-Wrap.

The material of the core cover (86) may comprise any known type of substrate, including nonwovens, webs, garments, textiles, films, tissues and laminates of two or more substrates or webs. The core cover material may comprise natural fibers, such as cellulose, cotton, flax, linen, hemp, wool, silk, fur, hair and naturally occurring mineral fibers. The core cover material may also comprise synthetic fibers such as rayon and lyocell (derived from cellulose), polysaccharides (starch), polyolefin fibers (polypropylene, polyethylene), polyamides, polyester, butadiene-styrene block copolymers, polyurethane and combinations there- of. Preferably, the core cover (86) comprises synthetic fibers or tissue.

The fibers may be mono- or multicomponent. Multicomponent fibers may comprise a homopolymer, a copolymer or blends thereof.

A schematic view of an inventive absorbent core (80) or so called absorbent paper is shown in Fig. 2.

According to the invention the absorbent core (80) comprises at least two thin and flexible single layers (91, 92) of suitable absorbent material. Each of these layers is macroscopically two-dimensional and planar and of very low

thickness compared to the other dimensions. Said layer may incorporate superabsorbent material throughout the layer.

The layers may have different concentrations and different water-absorbent polymer material showing concentrations in the range from about 90 to 100% by weight.

The layers (91, 92) are preferably joined to each e.g. by addition of adhesives (93) or by mechanical, thermal or ultrasonic bonding or combinations thereof, whereas adhesives are pre-ferred.

Furthermore it can be preferred that the water-absorbent polymer particles are placed within the core (80), especially within each layer (91, 92) in discrete regions, chambers or pockets,

e.g. supported by at least an adhesive.

Techniques of application of the water-absorbent polymer materials into the absorbent core es-pecially the respective layers (91, 92) are known to persons skilled in the art and may be volumetric, loss-in-weight or gravimetric. Known techniques include the application by vibrating systems, single and multiple auger systems, dosing roll, weigh belt, fluid bed volumetric systems and gravitational sprinkle and/or spray systems. Further techniques of insertion are falling dosage systems consensus and contradictory pneumatic application or vacuum printing method of applying the fluid absorbent polymer materials.

The quantity of water-absorbent polymer particles within the fluid-absorbent core (80) (absorbent paper) is from 100 to 500 gsm, preferably 200 to 400gsm, more preferably 250 to 300 gsm in case of maxi diapers (size L), wherein each layer contains at least 50 gsm water absorbent polymer particles preferably at least 100 gsm water absorbent polymer particles

The absorbent core (80) may comprise also at least one layer of other material such as short-fiber air-laid nonwoven materials (94); nonwoven materials such as polyethylene, polypropylene, nylon, polyester, and the like; cellulosic fibrous materials such as paper tissue or towels known in the art, wax-coated papers, corrugated paper materials, and the like; or fluff pulp. Said layer may further incorporate bi-component binding fibers.

The nonwoven (94) within in the absorbent core (80) is typically a single layer, e.g. made by air-thru bonded process. Its total basis weight is around 10 to 100 gsm, preferably 40 to 60.

The absorbent core (80) additionally may comprise at least two tissue layers (95, 96). The tis-sue layers are not restricted to tissue material such as paper it also refers to nonwovens.

The material of the layers (95, 96) may comprise any known type of substrate, including webs, garments, textiles and films. The tissue layers (95, 96) may comprise natural fibers, such as cellulose, cotton, flax, linen, hemp, wool, silk, fur, hair and naturally occurring mineral fibers. The tissue layer (95, 96) may also comprise synthetic fibers such as rayon and lyocell (de-rived from cellulose), polysaccharides (starch), polyolefin fibers (polypropylene, polyethylene), polyamides, polyester, butadiene-styrene block copolymers, polyurethane and combinations thereof. Preferably, the tissue layer comprises cellulose fibers. It is preferred that the tissue layer is made from ca. 50% wood pulp and 50% chemical viscose fibers at >45 gsm to provide tensile strength and integrity.

According to the invention the upper and lower tissue layers (95, 96) each total basis weight is from 10 to 100 gsm, preferably 30 to 80 gsm.

An absorbent core (80) according to the invention comprises at least two layers of water-absorbent polymer particles one of which laid on top side (91) and another laid on the bottom (92). Both layers are connected (93) with e.g. adhesives, ultrasonic bonding and/or heat bonding on e.g. air-thru-bond nonwoven material (94) which is sandwiched by the two layers (91, 92). For a good core integrity the upper sheet (tissue layer) (95) and/or the lower sheet (tissue layer) (96) are joined to the surface of the upper layer of water-absorbent polymer particles (91) and the lower layer of water-absorbent polymer particles (92) respectively.

According to the invention it is preferred that the fluid-absorbent core (80) comprises not more than 20% by weight of an adhesive, preferably not more than 10% by weight of an adhesive. Preferably the adhesive is a hotmelt adhesive

The absorbent core (80) respectively has a total basis weight ranging from about 150 gsm to about 2000 gsm, preferably from about 300 gsm to about 750 gsm, and more preferrably from about 500 gsm to about 650 gsm.

According to the invention at least one of the layers (91) and/or (92) containing a blend of at least two kinds of water-absorbent polymer particles.

It is preferred that the upper layer (91) containing a blend of the water-absorbent polymer particles G with water-absorbent particles H.

According to the invention it is preferred that the upper layer (91) contains 10 to 50% by weight of the water-absorbent polymer particles G based on the total sum of water-absorbent polymer particles, preferably 15 to 40% by weight of the water-absorbent polymer particles G, more preferably 20 to 35% by weight of the water-absorbent polymer particles G.

According to the invention it is preferred that the upper layer (91) contains 90 to 50% by weight of the water-absorbent polymer particles H based on the total sum of water-absorbent polymer particles, preferably 85 to 60% by weight of the water-absorbent polymer particles H, more preferably 80 to 65% by weight of the water-absorbent polymer particles H.

Preferably, the water absorbent mixture within the upper layer (91) comprises 50 to 90 wt.-% of water-absorbent polymer particles H and 50 to 10 wt.-% of water-absorbent polymer particles G. More preferably the mixture or blend comprises 60 to 85 wt.-% of the water-absorbent polymer particles H and 40 to 15 wt.-% of water-absorbent polymer particles G. Most preferably the mixture comprises 65 to 80 wt.-% of the water-absorbent polymer particles H and 35 to 20 wt.-% of water-absorbent polymer particles G.

The water-absorbent polymer particles H are preferably irregular particles.

The water-absorbent polymer particles H, having a CRC of at least 30 g/g, preferably of at least 35 g/g.

According to a further object of the invention it is preferred that also the water-absorbent polymer particles of the lower layer (92) are any type of water-absorbent polymer particles.

According to the invention the water-absorbent polymer particles are surface-crosslinked.

According to the invention the lower layer (92) containing at least one kind of water-absorbent polymer particles.

One preferred fluid-absorbent core (80) according to the invention comprises within the lower layer (92) water-absorbent polymer particles with a CRC of at least 30g/g and a vortex of at least 30 s.

According to the invention it is more preferred that the water-absorbent polymer particles of the lower layer (92) have a CRC of at least 32 g/g and a vortex of 40 s.

According to the invention it is most preferred that the water-absorbent polymer particles of the lower layer (92) have a CRC of at least 35 g/g and a vortex of 50 s.

Vortex and CRC could be e.g. adapted by modifying crosslink density or particle size distribution of the water-absorbent polymer particles.

According to a further preferred embodiment the upper layer (91) and/or the lower layer (92) comprises 100% by weight of water-absorbent particles.

In another embodiment a fluid-absorbent core (80) the water-absorbent particles are placed in discrete regions within at least one layer (91, 92) of the fluid-absorbent core (80).

According to a further embodiment a nonwoven material (94) is sandwiched between the upper layer (91) and the lower layer (92).

It is preferred that the water-absorbent polymer particles of the lower layer (92) show higher Vortex than the blend of water-absorbent polymers G and H within the upper layer (91). The vortex of the water-absorbent polymer particles within the lower layer is at least 40 s, preferably 45 s, more preferably 50 s.

But it is also possible that the lower layer comprises a blend of at least two different water-absorbent polymer particles showing a vortex of at least 40 s, preferably 45s, more preferably 50s.

The inventive absorbent core ensures by the combination of different water-absorbent polymers in the upper and lower layer, including blends respectively, a fast absorption of the insulted fluid, especially by the upper layer and a storage of the fluid later on in the lower layer.

The blend of water-absorbent polymer particles G showing fast water absorption with water-absorbent polymer particles H having a vortex above 50s, or preferably above 60 s, more preferably above 70 s ensures a fast liquid absorption and low rewet.

Especially in combination with a lower layer (92) comprising water absorbent polymer particles having a vortex of at least 40 s the absorbent core according to the invention surprisingly ensures a fast fluid absorption, preventing leakage, and a high capacity and low rewet, ensuring dryness for a wearer.

In one embodiment of the inventive absorbent paper/absorbent core the upper layer (91) comprises 100% by weight of water-absorbent particles and/or the lower layer (92) comprises 100% by weight of water-absorbent particles.

The inventive absorbent core not only absorbs fluids fast with a low rewet, ensuring not only less leaking but also dryness for a wearer, it furthermore provides a high capacity for liquids as shown e.g. by the TAC $_{AP,30\ min}$ above 500 g/g and a CRC $_{AP,\ 30min}$ of above 320 g/g respectively.

According to the invention a preferred absorbent core (80) comprises top (91) and bottom (92) layers of water-absorbing polymer particles containing each 130 grams per square meter (g/m$^2$). Both layers are glued (93) with 0.5 g/m$^2$ hot melt adhesive on 50 g/m$^2$ air-thru-bond nonwoven material (94) and are then sandwiched with two layers of 45 g/m$^2$ condensed tissue layers on the top (95) and bottom (96) using hot-melt glue applied to the surface at 2.0 g/m$^2$. Total hot-melt glue used is 2.5 g/m$^2$ for both top and bottom layers

The density of the fluid-absorbent core is in the range of 0.1 to 0.25 g/cm$^3$, preferably 0.1 to 0.28 g/cm$^3$. The thickness of the fluid-absorbent core is in the case of diapers in the range of 1 to 8 mm, preferably 1 to 5 mm, more preferably 1.5 to 3 mm, in the case of adult- incontinence products in the range of 3 to 15 mm.

The TAC $_{AP,30\ min}$ and of the fluid absorbent core (80) according to the invention is above 500 g/g, preferably above 520 g/g and the CRC $_{AP,\ 30min}$ of the fluid absorbent core (80) are above 320 g/g, preferably above 340 g/g, Fig. 3 illustrates a possible production process for an absorbent core.

A first water-absorbent polymer or a blend of water absorbent polymers (230) is dropped onto one side of a nonwoven material (250). Then an adhesive (200) is applied to the top tissue paper (210). The tissue paper (210) then is laminated with the side of the nonwoven (250) carrying the water-absorbent polymer (230). Afterwards the nonwoven

(250) is turned around and the second water- absorbent polymer (240) is dropped onto the other side of the nonwoven (250). An adhesive (200) is applied to the bottom or lower tissue paper (260). The tissue paper (260) then is laminated with the side of the nonwoven (250) carrying the water-absorbent polymer (240). Finally the absorbent paper is slit to the desired width and winding.

The fluid-absorbent core (80) typically has a uniform size or profile. Suitable fluid-absorbent cores can also have profiled structures, concerning the shape of the core and/or the content of water-absorbent polymer particles and/or the distribution of the water-absorbent polymer parti-cles and/or the dimensions of the different layers if a layered fluid-absorbent core is present.

The shape of the core in view from above (x-y dimension) can be rectangular, anatomical shaped with a narrower crotch area or any other shapes.

The top view area of the fluid-absorbent core (80) is preferably at least 200 $cm^2$, more preferably at least 250 $cm^2$, most preferably at least 300 $cm^2$. The top view area is the part of the core that is face-to-face to the upper liquid-pervious layer.

The fluid-absorbent core may comprise additional additives typically present in fluid absorbent articles known in the art. Exemplary additives are fibers for reinforcing and stabilizing the fluid-absorbent core. Preferably polyethylene is used for reinforcing the fluid- absorbent core.

Further suitable stabilizer for reinforcing the fluid-absorbent core are materials acting as binder.

In varying the kind of binder material or the amount of binder used in different regions of the flu-id-absorbent core it is possible to get a profiled stabilization. For example, different binder mate-rials exhibiting different melting temperatures may be used in regions of the fluid-absorbent core, e.g. the lower melting one in the central region of the core, and the higher melting in the distal regions.

Suitable binder materials may be adhesive or non-adhesive fibers, continuously or discontinuously extruded fibers, bi-component staple fibers, non- elastomeric fibers and sprayed liquid binder or any combination of these binder materials.

Further, thermoplastic compositions usually are added to increase the integrity of the core lay-er. Thermoplastic compositions may comprise a single type of thermoplastic polymers or a blend of thermoplastic polymers. Alternatively, the thermoplastic composition may comprise hot melt adhesives comprising at least one thermoplastic polymer together with thermoplastic diluents such as tackifiers, plasticizers or other additives, e.g. antioxidants. The thermoplastic composition may further comprise pressure sensitive hot melt adhesives comprising e.g. crystalline polypropylene and an amorphous polyalphaolefin or styrene block copolymer and mixture of waxes.

Concerning odor control, perfumes and/or odor control additives are optionally added. Suitable odor control additives are all substances of reducing odor developed in carrying fluid-absorbent articles over time known in the art. Thus, suitable odor control additives are inorganic materials, such as zeolites, activated carbon, bentonite, silica, aerosile, kieselguhr, clay; chelants such as ethylenediamine tetraacetic acid (EDTA), cyclodextrins, aminopolycarbonic acids, ethylenediamine tetramethylene phosphonic acid, aminophosphate, polyfunctional aromates, N,N-disuccinic acid. Suitable odor control additives are further antimicrobial agents.

Suitable odor control additives are further compounds with anhydride groups such as maleic-, itaconic-, polymaleic- or polyitaconic anhydride, copolymers of maleic acid with C2-C8 olefins or styrene, polymaleic anhydride or copolymers of maleic anhydride with isobutene, di-isobutene or sty-rene, compounds with acid groups such as ascorbic, benzoic, citric, salicylic or sorbic acid and fluid-soluble pol-ymers of monomers with acid groups, homo- or copolymers of C3-C5 mono-unsaturated carboxylic acids.

Newest developments propose the addition of wetness indication additives.

Suitable wetness indication additives comprising a mixture of sorbitan monooleate and polyethoxylated hydrogenated castor oil. Preferably, the amount of the wetness indication addi-tive is in the range of about 0.0001 to 2 % by weight related to the weight of the fluid- absorbent core.

Acquisition-distribution Layer (70)

An optional acquisition-distribution layer (70) is located between the upper layer (A) (89) and the fluid-absorbent core (80) and is preferably constructed to efficiently acquire discharged body fluids and to transfer and distribute them to other regions of the fluid- absorbent composition or to other layers, where the body fluids are immobilized and stored. Thus, the upper layer transfers the discharged liquid to the acquisition-distribution layer (D) for distributing it to the flu-id-absorbent core.

The acquisition-distribution layer (70) comprises fibrous material and optionally water- absorbent polymer particles.

The fibrous material may be hydrophilic, hydrophobic or can be a combination of both hydrophilic and hydrophobic fibers. It may be derived from natural fibers, synthetic fibers or a combination of both.

Suitable acquisition-distribution layers are formed from cellulosic fibers and/or modified cellu-losic fibers and/or

synthetics or combinations thereof. Thus, suitable acquisition-distribution layers may contain cellulosic fibers, in particular wood pulp fluff. Examples of further suitable hydrophilic, hydrophobic fibers, as well as modified or unmodified natural fibers are given in the chapter " Liquid-pervious sheet or liquid pervious layer (89)" above.

Especially for providing both fluid acquisition and distribution properties, the use of modified cellulosic fibers are preferred. Examples for modified cellulosic fibers are chemically treated cellulosic fibers, especially chemically stiffened cellulosic fibers. The term "chemically stiffened cellulosic fibers" means cellulosic fibers that have been stiffened by chemical means to increase the stiffness of the fibers. Such means include the addition of chemical stiffening agent in the form of surface coatings, surface cross-linking and impregnates. Suitable polymeric stiffening agents can include: cationic modified starches having nitrogen-containing groups, latexes, wet strength resins such as polyamide-epichlorohydrin resin, polyacrylamide, urea formaldehyde and melamine formaldehyde resins and polyethylenimine resins

Stiffening may also include altering the chemical structure, e.g. by crosslinking polymer chains. Thus crosslinking agents can be applied to the fibers that are caused to chemically form intrafiber crosslink bonds. Further cellulosic fibers may be stiffened by crosslink bonds in individualized form. Suitable chem-ical stiffening agents are typically monomeric crosslink- ing agents including C2-C8 dialdehyde, C2-C8 monoaldehyde having an acid functionality, and especially C2-C9 polycarboxylic acids.

Stiffening may also include altering the chemical structure, e.g. by crosslinking polymer chains. Thus crosslinking agents can be applied to the fibers that are caused to chemically form intrafiber crosslink bonds. Further cellulosic fibers may be stiffened by crosslink bonds in individualized form. Suitable chem-ical stiffening agents are typically monomeric crosslink- ing agents including C2-C8 dialdehyde, C2-C8 monoaldehyde having an acid functionality, and especially C2-C9 polycarboxylic acids.

Preferably the modified cellulosic fibers are chemically treated cellulosic fibers. Especially preferred are curly fibers which can be obtained by treating cellulosic fibers with citric acid. Preferebly the basis weight of cellulosic fibers and modified cellulosic fibers is from 50 to 200 gsm.

Suitable acquisition-distribution layers further include synthetic fibers. Known examples of syn-thetic fibers are found in the Chapter " Liquid-pervious sheet or liquid pervious layer (89)" above. Another possibility available is 3D-polyethylene film with dual function as a liquid-pervious layer (89) and acquisition-distribution layer.

Further, as in the case of cellulosic fibers, hydrophilic synthetic fibers are preferred. Hydrophilic synthetic fibers may be obtained by chemical modification of hydrophobic fibers. Prefer-ably, hydrophilization is carried out by surfactant treatment of hydrophobic fibers. Thus the sur-face of the hydrophobic fiber can be rendered hydrophilic by treatment with a nonionic or ionic surfactant, e.g., by spraying the fiber with a surfactant or by dipping the fiber into a surfactant. Further preferred are permanent hydrophilic synthetic fibers.

The fibrous material of the acquisition-distribution layer may be fixed to increase the strength and the integrity of the layer. Technologies for consolidating fibers in a web are mechanical bonding, thermal bonding and chemical bonding. Detailed description of the different methods of increasing the integrity of the web is given in the Chapter " Liquid-pervious sheet or liquid pervious layer (89)" above.

Preferred acquisition-distribution layers comprise fibrous material and water-absorbent polymer particles distributed within. The water-absorbent polymer particles may be added during the process of forming the layer from loose fibers, or, alternatively, it is possible to add monomer solution after the formation of the layer and polymerize the coating solution by means of UV-induced polymerisation technologies. Thus, "in situ"-polymerisation is a further method for the application of water-absorbent polymers.

Thus, suitable acquisition-distribution layers comprising from 80 to 100% by weight a fibrous material and from 0 to 20% by weight water-absorbent polymer particles; preferably from 85 to 99.9% by weight a fibrous material and from 0.1 to 15% by weight water- absorbent polymer particles; more preferably from 90 to 99.5% by weight a fibrous material and from 0.5 to 10% by weight water-absorbent polymer particles; and most preferably from 95 to 99% by weight a fibrous material and from 1 to 5% by weight water-absorbent polymer particles

Alternatively a liquid-impervious layer (70) comprising a synthetic resin film between (89) and (80) acting as an distribution layer and quickly transporting the supplied urine along the surface to the upper lateral portion of the fluid-absorbent core (80). Preferably, the upper liquid-impervious layer (70) is smaller than the underlaying fluid-absorbent core (80). There is no limit in particular to the material of the liquid-impervious layer (70). Such a film made of a resin such as polyethylene, polypropylene, polyethylene therephthalate, polyurethane, or crosslinked polyvinyl alcohol and an air-permeable, but liquid-impervious, so- called: "breathable" film made of above described resin, may be used.

Preferably, the upper liquid-impervious layer (70) comprises a porous polyethylene film for both quick acquisition and distribution of fluid.

Alternatively a bundle of synthetic fibers acting as acquisition-distribution layer loosely distributed on top of the fluid-absorbent core may be used. Suitable synthetic fibers are of copolyester, polyamide, copolyamide, polylactic acid, polypropylene or polyethylene, viscose or blends thereof. Further bicomponent fibers can be used. The synthetic fiber component may be composed of either a single fiber type with a circular cross-section or a blend of two fibre types with

different cross- sectional shapes. Synthetic fibers arranged in that way ensuring a very fast liquid transport and canalisation. Preferably bundles of polyethylene fibers are used.

Other Optional Components (F)

1. Leg Cuff

Typical leg cuffs comprising nonwoven materials which can be formed by direct extrusion pro-cesses during which the fibers and the nonwoven materials are formed at the same time, or by laying processes of preformed fibers which can be laid into nonwoven materials at a later point of time. Examples for direct extrusion processes include spunbonding, melt-blowing, solvent spinning, electrospinning and combinations thereof. Examples of laying processes include wet-laying and dry-laying (e.g. air-laying, carding) methods. Combinations of the processes above include spunbond-meltblown-spunbond (sms), spunbond-meltblow-meltblown-spunbond (smms), spunbond-carded (sc), spunbond-airlaid (sa), meltblown-airlaid (ma) and combinations thereof. The combinations including direct extrusion can be combined at the same point in time or at a subsequent point in time. In the examples above, one or more individual layers can be produced by each process. Thus, "sms" means a three layer nonwoven material, "smsms" or "ssmms" means a five layer nonwoven material. Usually, small type letters (sms) designate individual layers, whereas capital letters (SMS) designate the compilation of similar adjacent layers.

Further, suitable leg cuffs are provided with elastic strands.

Preferred are leg cuffs from synthetic fibers showing the layer combinations sms, smms or smsms. Preferred are nonwovens with the density of 13 to 17 gsm. Preferably leg cuffs are provided with two elastic strands.

2. Elastics

The elastics are used for securely holding and flexibly closing the fluid-absorbent article around the wearers' body, e.g. the waist and the legs to improve containment and fit. Leg elas-tics are placed between the outer and inner layers or the fluid-absorbent article, or between the outer garment facing cover and the user facing bodyside liner. Suitable elastics comprising sheets, ribbons or strands of thermoplastic polyurethane, elastomeric materials, poly(ether-amide) block copolymers, thermoplastic rubbers, styrene-butadiene copolymers, silicon rubbers, natural rubbers, synthetic rubbers, styrene isoprene copolymers, styrene ethylene butylene copolymers, nylon copolymers, spandex fibers comprising segmented polyurethane and/or ethylene-vinyl acetate copolymer. The elastics may be secured to a substrate after being stretched, or secured to a stretched substrate. Otherwise, the elastics may be secured to a substrate and then elastisized or shrunk, e.g. by the application of heat.

3. Closing System

The closing system can include tape tabs, landing zone, elastomerics, pull ups and the belt system or combinations thereof.

At least a part of the first waist region is attached to a part of the second waist region by the closing system to hold the fluid-absorbent article in place and to form leg openings and the waist of the fluid-absorbent article. Preferably the fluid-absorbent article is provided with a re-closable closing system.

The closing system is either re-sealable or permanent, including any material suitable for such a use, e.g. plastics, elastics, films, foams, nonwoven substrates, woven substrates, paper, tissue, laminates, fiber reinforced plastics and the like, or combinations thereof. Preferably the closing system includes flexible materials and works smooth and softly without irritating the wearer ' s skin

One part of the closing elements is an adhesive tape, or comprises a pair of laterally extending tabs disposed on the lateral edges of the first waist region. Tape tabs are typically attached to the front body panel and extend laterally from each corner of the first waistband. These tape tabs include an adhesive inwardly facing surface which is typically protected prior to use by a thin, removable cover sheet.

Suitable tape tabs may be formed of thermoplastic polymers such as polyethylene, polyurethane, polystyrene, polycarbonate, polyester, ethylene vinyl acetate, ethylene vinyl alcohol, eth-ylene vinyl acetate acrylate or ethylene acrylic acid copolymers.

Suitable closing systems comprise further a hook portion of a hook and loop fastener and the target devices comprise the loop portion of a hook and loop fastener.

Suitable mechanical closing systems including a landing zone. Mechanical closing systems may fasten directly into the outer cover. The landing zone may act as an area of the fluid-ab-sorbent article into which it is desirable to engage the tape tabs. The landing zone may include a base material and a plurality of tape tabs. The tape tabs may be embedded in the base material of the landing zone. The base material may include a loop material. The loop material

may include a backing material and a layer of a nonwoven spunbond web attached to the backing material.

Thus suitable landing zones can be made by spunbonding. Spunbonded nonwoven are made from melt-spun fibers formed by extruding molten thermoplastic material. Preferred is bi-oriented polypropylene (BOPP), or brushed/closed loop in the case of mechanical closing systems.

Further, suitable mechanical closing systems including elasticomeric units serving as a flexible abdominal and/or dorsal discrete waist band, flexible abdomen and/or dorsal zones located at distal edge for fluid-absorbents articles, such as pants or pull-ups. The elasticomeric units enable the fluid-absorbent article to be pulled down by the wearer as e.g. a training pant.

Suitable pants-shaped fluid-absorbent article has front abdominal section, rear dorsal section, crotch section, side sections for connecting the front and rear sections in lateral direction, hip section, elastic waist region and liquid-tight outer layer. The hip section is arranged around the waist of the user. The disposable pants-shaped fluid-absorbent article (pull-up) has favorable flexibility, stretchability, leak-proof property and fit property, hence imparts excellent comfort to the wearer and offers improved mobility and discretion

Suitable pull-ups comprising thermoplastic films, sheets and laminates having a low modulus, good tear strength and high elastic recovery.

Suitable closing systems may further comprise elastomerics for the production of elastic areas within the fastening devices of the fluid-absorbent article. Elastomerics provide a conformable fit of the fluid-absorbent article to the wearer at the waist and leg openings, while maintaining adequate performance against leakage.

Suitable elastomerics are elastomeric polymers or elastic adhesive materials showing vapor permeability and liquid barrier properties. Preferred elastomerics are retractable after elongation to a length equivalent to its original length.

Suitable closing systems further comprise a belt system, comprising waist-belt and leg-belts for flexibly securing the fluid-absorbent article on the body of the wearer and to provide an improved fit on the wearer. Suitable waist-belts comprising two elastic belts, a left elastic belt, and a right elastic belt. The left elastic belt is associated with each of the left angular edges. The right elastic belt associated with each of the right angular edges. The left and right side belts are elastically extended when the absorbent garment is laid flat.

Each belt is connected to and extends between the front and rear of the fluid-absorbent article to form a waist hole and leg holes.

Preferably the belt system is made of elastomerics, thus providing a conformable fit of the flu-id-absorbent article and maintaining adequate performance against leakage.

Preferred closing systems are so-called "elastic ears" attached with one side of the ear to the longitudinal side edges located at the rear dorsal longitudinal edge of the chassis of the fluid-absorbent article. Commercially available fluid-absorbent articles include stretchable ears or side panels which are made from a stretchable laminate e.g. nonwoven webs made of mono- or bi-component fibers. Especially preferred closing systems are stretchable laminates comprising a core of several layers each of different fibrous materials, e.g. meltblown fibers, spunbond fibers, containing multicomponent fibers having a core comprising a first polymer having a first melt temperature and a sheath comprising a second polymer having a second melt temperature; and a web of an elastomeric material as top and bottom surfaces to form said laminate.

C. Fluid-absorbent article Construction

The present invention further relates to the joining of the components and layers, films, sheets, tissues or substrates mentioned above to provide the fluid-absorbent article. At least two, preferably all layers, films, sheets, tissues or substrates are joined.

Suitable fluid-absorbent articles include a single- or multiple fluid-absorbent core-system. Preferably fluid-absorbent articles include a single- or double fluid-absorbent core-system Suitable fluid-storage layers of the fluid-absorbent core (80); comprising 0 to 20% by weight fibrous material and from 80 to 100% by weight a water-absorbent polymer material, preferably comprising from 0 to 10% by weight a fibrous material and from 90 to 100% by weight water-absorbent polymer particles; more preferably from 0 to 5% by weight a fibrous material and from 95 to 100% by weight water-absorbent polymer particles. In case of fibrous material present, the fibrous materials homogeneously or in-homogeneously mixed with water-absorbent polymer particles. Suitable fluid-storage layers of the fluid-absorbent core including a layered fluid-absorbent core-system comprising 100% by weight of water-absorbent polymer material or homogeneous or in-homogeneous mixtures of fibrous materials and water-absorbent polymer particles.

In order to immobilize the water-absorbent polymer particles, the adjacent layers are fixed by the means of thermoplastic materials, thereby building connections throughout the whole sur-face or alternatively in discrete areas of junction. For the latter case, cavities or pockets are built carrying the water-absorbent particles. The areas of junction may have a regular or irregular pattern, e.g. aligned with the longitudinal axis of the fluid-absorbent core or in a pattern of polygons, e.g. pentagons or hexagons. The areas of junction itself may be of rectangular, circular or squared shape with diameters between about 0.5 mm and 2 mm. Fluid-absorbent articles comprising areas of junction show a better

wet strength.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

In order to ensure wicking of applied body fluids, preferred fluid-absorbent article show channels for better transport. Channels are formed by compressional forces of e.g. the top sheet against the fluid-absorbent core. Compressive forces may be applied e.g. by heat treatment between two heated calendar rollers. As an effect of compression both on top sheet and fluid-absorbent core deform such that a channel is created. Body fluids are flowing along this channel to places where they are absorbed and leakage is prevented. Otherwise, compression leads to higher density; this is the second effect of the channel to canalize insulted fluids. Additionally, compressive forces on diaper construction improve the structural integrity of the fluid-absorbent article.

Typically fluid-absorbent articles comprising at least an upper liquid-pervious layer (89), at least a lower liquid-impervious layer (83) and at least one fluid-absorbent core (80) between
the layer (89) and the layer (83) besides other optional layers.

The inventive fluid-absorbent article shows improved rewet and fluid acquisition properties.

A fluid absorbent article according to the invention, comprising

(A) an upper liquid-pervious sheet (89),
(B) a lower liquid-impervious sheet (83),
(C) a fluid-absorbent core (80)
(D) an acquisition-distribution layer (70) between the upper liquid-pervious sheet (89) and the fluid-absorbent core (80),
(F) other optional components,

whereas the fluid-absorbent core (80) comprising at least two layers, an upper layer (91) and a lower layer (92), each layer comprising from 0 to 10% by weight fibrous material and from 90 to 100% by weight water-absorbent polymer particles, based on the sum of water-absorbent polymer particles and fibrous material,
wherein within the upper layer (91) a water absorbent mixture comprising at least 10% by weight of a first type of water-absorbent polymer particles, water-absorbent polymer particles G with a vortex of less than 30s and at least 50% by weight of a second type of water-absorbent polymer particles, water-absorbent polymer particles H, with a CRC of at least 25 g/g wherein water-absorbent polymer particles G are obtained by agglomerating fine water-absorbing polymer particles by using a solution or suspension comprising

a) 0,04 to 1,2 % by weight water-soluble or water-dispersible polymeric binders or a mixture thereof, based on the water-absorbent polymer particles,
b) 20 to 70% by weight of water based on the water-absorbent polymer particles, and
c) 5 to 20% by weight of a water-miscible organic solvent based on the water-absorbent polymer particles

and wherein fine water-absorbing polymer particles having an average particle diameter of not larger than 300 $\mu$m.

Furthermore according to another embodiment the fluid absorbent article has a water pouring time of less than 35 s and a rewet value of less than 0,8 g, preferably the fluid absorbent article has a water pouring time of less than 33 s and a rewet value of less than 0,8 g, more preferably the fluid absorbent article has a water pouring time of less than 32 s and a rewet value of 0,7 g or less.

The inventive fluid absorbent article preferably has a TAC $_{AP,30\ min}$ of more than 500 g/g.

Methods:

The measurements should, unless stated otherwise, be carried out at an ambient temperature of 23 $\pm$ 2° C and a relative atmospheric humidity of 50 $\pm$ 10%. The water-absorbent polymers are mixed thoroughly before the measurement.

The "WSP" standard test methods are described in: "Standard Test Methods for the Nonwovens Industry", jointly issued by the "Worldwide Strategic Partners" EDANA (European Disposables and Nonwovens Association, Avenue Eugene Plasky, 157, 1030 Brussels, Belgium, www.edana.org) and INDA (Association of the Nonwoven Fabrics Industry, 1100 Crescent Green, Suite 115, Cary, N.C. 27518, U.S.A., www.inda.org). This publication is available both from EDANA and INDA

Absorbency Under Load (0.3psi)

The absorbency under load (AUL or AAP) of the water-absorbent polymer particles is determined analogously to the EDANA recommended test method No. WSP 242.3 (11) "Gravimetric Determination of Absorption Under Pressure".

Bulk Density

The bulk density of the water-absorbent polymer particles is determined by the EDANA recommended test method No. WSP 250.3 (11) "Gravimetric Determination of Density"

Centrifuge Retention Capacity (CRC)

The centrifuge retention capacity of the water-absorbent polymer particles is determined by the EDANA recommended test method No. WSP 241.3 (11) "Fluid Retention Capacity in Saline, After Centrifugation", wherein for higher values of the centrifuge retention capacity larger tea bags have to be used.

Particle Size Distribution (PSD)

The particle size distribution of the water-absorbent polymer particles is determined by the EDANA recommended test method No. WSP 220.3 (11) "Particle Size Distribution".

The average particle diameter or also referred to as mean particle size ($d_{50}$) here is the value of the mesh size which gives rise to a cumulative 50% by weight.

The degree of polydispersity $\alpha$ of the particle size particle is calculated by

$$\alpha = (d_{84.13} - d_{15,87})/(2 \text{ x } d_{50})$$

wherein $d_{15,87}$ and $d_{84,13}$ is the value of the mesh size which gives rise to a cumulative 15,87% respective 84,13% by weight.

Vortex

The vortex time represents an absorption speed of water-absorbent particles at 0.9 mass % of saline under stirring. Pipe 50.0 ± 1.0 ml of 0.9% NaCl solution into a 100 mL beaker with a stir bar stirring on magnetic stirring plate at a rotation speed of 600 rpm. The liquid surface produces a vortex under stirring. Then 2.000 ± 0.010 gram of water-absorbent particles are weighted and added into the beaker as quickly as possible, with starting the stop watch synchronously. The stopwatch is stopped when the surface of liquid becomes "still", namely, the surface has no turbulence while the mixture may still be turning. The displayed time (in seconds) is recorded as the vortex time.

Test methods of laminates

Water pouring time and rewet test

This test consists of multiple insults of deionized water. The rewet is measured by the amount of fluid the article released under pressure.

The test specimen (fluid-absorbent article or absorbent core respectively) is put into a transparent test box with length of 41.0 cm, width of 12.0 cm and height of 6.0 cm. Measure 500 ml of Grade 2 water having conductivity at 25 ± 1° C temperature of 0.1 Ms/m according to the EN ISO 3696:1995 using 1000 ml plastic beaker. Pour the water onto the specimen into the test box back and forth within 15 s, once the water first contacts the specimen, simultaneously start a stopwatch. When the water is visually absorbed by the specimen, stop the stopwatch and record the "water pouring time" in seconds. Allow the test specimen to fully absorb the water for 30 seconds, monitored by a count-down timer. Prepare 30-40 pieces of filter paper (MN 615, OD 9 cm) and record its dry weight in grams (DW1). After 30 seconds, place the filter paper on to the test specimen at the center and place 2.47 kg circular weight with OD 8 cm on top the filter paper. Wait for 30 seconds monitored by a timer, then take out the weight and the filter paper. Weigh the filter and record its wet weight in grams ($WW_1$).

$$\text{Rewet (g)} = WW_1 \text{ (g)} - DW_1 \text{ (g)}$$

$TAC_{AP,\ 30\ min}$

Total absorption capacity 30 min ($TAC_{AP,\ 30\ min}$)

Total absorption capacity (TAC) measures the ability of the absorbent paper absorbs 0.9% saline solution for 30 ± 1 minutes. The specimen is wrapped with nonwoven sheet to prevent SAP lost during the test and weigh its dry weight (WD). The sample is then submerged into 0.9% saline solution for 30 minutes respectively. After that, it is hanged a bar with the sample centerline for 2 minutes to drain out excess liquid. Then its wet-weight (WW) is recorded.

$$\text{Total Absorption Capacity } (TAC_{AP,\ 30min},\ g) = WW(g) - WD(g)$$

$CRC_{AP,30\ min}$

The specimen is wrapped with nonwoven sheet to prevent SAP lost during the test and weigh its dry weight (WD). The sample is then submerged into 0.9% saline solution for 30 minutes. After that, it is hanged a bar with the sample centerline for 2 minutes to drain out excess liquid. Then the sample is placed in a spin dryer (with speed of 1400 rpm) and spin dry for 3 minutes. After spinning, weight its weight (WS).

$$\text{Centrifuge Retention Capacity of the absorbent paper } (CRC_{AP,30\ min},\ g) = WS(g) - WD(g)$$

The EDANA test methods are obtainable, for example, from the EDANA, Avenue Eugène Plasky 157, B-1030 Brussels, Belgium

Example 1

Base fine water-absorbent polymer particles

By continuously mixing water, 50% by weight sodium hydroxide solution and acrylic acid, a 42.7% by weight acrylic acid/sodium acrylate solution was prepared such that the degree of neutralization was 69.0 mol%. After the components had been mixed, the monomer solution was cooled continuously to a temperature of 30 °C by means of a heat exchanger and degassed with nitrogen. The polyethylenically unsaturated crosslinker used was 3-tuply ethoxylated glyceryl triacrylate (purity approx. 85% by weight). The amount used, based on the acrylic acid (boaa) used, 0.35% by weight. To initiate the free-radical polymerization, the following components were used: 0.002% by weight boaa of hydrogen peroxide, metered in as a 2.5% by weight aqueous solution, 0.1% by weight boaa of sodium peroxodisulfate, metered in as a 15% by weight aqueous solution, and 0.01% by weight boaa of ascorbic acid, metered in as a 0.5% by weight aqueous solution. The throughput of the monomer solution was 40 kg/h.

The individual components were metered continuously into a List ORP 10 Contikneter continuous kneader reactor (List AG, Arisdorf, Switzerland).

The reaction solution had a feed temperature of 30 °C. The residence time of the reaction mixture in the reactor was approx. 15 minutes.

The polymer gel thus obtained was extruded with an SLRE 75R extruder ( Sela Maschinen GmbH; Harbke; Germany). The temperature of the polymer gel in the course of extrusion was 85°C. The perforated plate had 12 holes having a hole diameter of 8 mm. The thickness of the perforated plate was 16 mm. The ratio of internal length to internal diameter of the extruder (L/D) was 4. The specific mechanical energy (SME) of the extrusion was 26 kWh/t. The extruded polymer gel was distributed on metal sheets and dried at 175°C in an air circulation drying cabinet for 90 minutes. The loading of the metal sheets with polymer gel was 0.81 g/cm$^2$.

The dried polymer gel was ground by means of a one-stage roll mill (three milling runs. 1st milling run with gap width 1000 μm, 2nd milling run with gap width 600 μm and 3rd milling run with gap width 400 μm). The ground dried polymer gel with size less than 150 μm was collected as base fine water-absorbent polymer particles (non-surface post-crosslinked fine water-absorbent polymer particles). The property characterization was shown in table 1.

Example 2

Surface crosslinking process

1.2 kg of base fine water-absorbent polymer particles from example 1 was coated in Pflugschar M5 plowshare

mixer with heating jacket (Gebr. Loedige Maschinenbau GmbH; Paderborn, Germany) at 23 °C and a shaft speed of 200 revolutions per minute by means of a two-substance spray nozzle with 54.6 g of a mixture of 0.07% by weight of N-hydroxyethyl-2-oxazolidinone, 0.07% by weight of 1,3-propanediol, 0.7% by weight of propylene glycol, 2.27% by weight of a 22% by weight aqueous aluminum lactate solution, 0.448% by weight of a 0.9% by weight aqueous sorbitan monolaurate solution and 0.992% by weight of isopropanol, the percentages by weight each being based on base polymer fine from example 1.

After the spray application, the product temperature was increased to 185 °C and the reaction mixture was held at this temperature and a shaft speed of 50 revolutions per minute for 35 minutes. The resulting product was cooled to ambient temperature and the fraction with a particle size of less than 150 μm was collected as surface crosslinked (SXL) polymer fine (surface post-crosslinked fine water-absorbent polymer particles). The property characterization was shown in table 1.

Example 3

The base fine water-absorbent polymer particles from example 1 and the SXL fine water-absorbent polymer particles from example 2 were blended with a weight ratio of 3:1 to obtain a mixture polymer fines as example 3. The property characterization was shown in table 1.

Table 1. Property characterization of polymer fines

| Exp. | < 45 μm [%] | 45-150 μm [%] | > 200 μm [%] | Vortex [s] | CRC [g/g] | 0.3psi [g/g] |
|---|---|---|---|---|---|---|
| Example 1 | 23.3 | 71.5 | 4.3 | 14.8 | 32.4 | 7.3 |
| Example 2 | 21.1 | 70.0 | 8.7 | 21.8 | 11.5 | 15.7 |
| Example 3 | | | | 18.2 | 26.8 | 10.0 |

Example 4

Agglomeration process

600 g of the fine water-absorbent polymer particles from example 3 was heated to 50 ° C in the oven, then was put into a 50 ° C preheated Pflugschar M5 plowshare mixer (Gebr. Loedige Maschinenbau GmbH; Paderborn, Germany) with maximum rotation speed of 370 rpm. 235 g of D.I water, 2.5 g of Sokalan® CP45 (BASF SE, Ludwigshafen, Germany), 62 g of isopropanol and 1 g of Denacol® 810 (Ethylene glycol diglycidyl ether, Nagase Chemicals, Ltd., Osaka, Japan) were mixed as the binder solution and sprayed to the example 3 with a spraying speed of 45 ml/min, thereafter the mixture was stirred at a high speed for 3 minutes. Then the mixer was stopped and the obtained hydrogel was dried at 130 ° C for 1.5 hours, pulverized by a blender (Oster, United States), and finally sized to 106-850 μm. The property characterization was shown in table 2.

Example 5

The procedure was as example 4, except that 150 g of D.I water, 0.24 g Sokalan® CP45, 30 g of isopropanol were used for agglomeration. The property characterization was shown in table 2.

Example 6

The procedure was as example 4, except that 150 g of D. I water, 4.74 g Sokalan® CP45, 93.6 g of isopropanol were used for agglomeration. The property characterization was shown in table 2.

Example 7

The procedure was as example 4, except that 400 g of D. I water, 4.74 g Sokalan® CP45, 93.6 g of isopropanol were used for agglomeration. The property characterization was shown in table 2.

Table 2. Property characterization of agglomerated fine

| Exp. | CRC g/g | Vortex sec | AUL g/g | Bulk density g/ml | PSD % | |
|---|---|---|---|---|---|---|
| | | | | | < 150μm | > 850μm |
| Example 4 | 18.9 | 5.2 | 18.4 | 0.45 | 34.4 | 6.6 |
| Example 5 | 20.8 | 10.5 | 24.3 | 0.52 | 34.0 | 7.7 |
| Example 6 | 20.4 | 9.3 | 23.3 | 0.52 | 16.8 | 13.1 |
| Example 7 | 19.1 | 14 | 21.6 | 0.47 | 3.6 | 22.7 |

Examples 8a, 8 and 9

Blending process

800 g example 4 and 800 g Hysorb® N7059 (Superabsorbent, BASF-YPC Company Ltd, Nanjin, China) for example 8a, or 600 g of example 4 and 1400 g Hysorb® N7059 for example 8 or 200g of example 4 and 1800 g of Hysorb® N7059 for example 9 respectively were added into Pflugschar M5 plowshare mixer (Gebr. Loedige Maschinenbau GmbH; Paderborn, Germany) at room temperature, then the two water-absorbent polymer particles were mixed at a rotation speed of 150 rpm for 5 minutes. The final properties of the mixture were compared with superabsorbents (SAPs) from San-Dia Polymer Ltd. (Sanwet IM 930NP) and Sumitomo Seika Chemicals Co., Ltd. (Aquakeep SA60SX-II) as shown in the table 3.

Table 3. CRC and Vortex of different SAPs

| Exp. | Description | CRC g/g | Vortex sec. | Calculated CRC g/g |
|---|---|---|---|---|
| Sanwet-IM930NP, San-Dia Polymer Ltd. | -- | 30.2 | 36.0 | -- |
| Aquakeep SA60SXII, Sumitomo Seika Chemicals Co., Ltd. | -- | 35.6 | 50.0 | -- |
| Hysorb®N 7059 | -- | 38.6 | 76.0 | -- |
| Example 8a | Example 4 : Hysorb®N7059 = 1:1 | 26.4 | 10.6 | 28.8 |
| Example 8 | Example 4 : Hysorb®N7059 = 3:7 | -- | -- | 32.7 |
| Example 9 | Example 4 : Hysorb®N7059 = 1:9 | -- | -- | 36.6 |

Example 10:

Preparation of absorbent paper samples

Hot melt glue (2.0 gsm) was sprayed on to tissue bottom layer (45 gsm), SAP (bottom layer) was then applied on to the tissue at 130 gsm loading using roller feeder. High loft nonwoven material (50 gsm) was fed into the lamination equipment, hot melt glue was sprayed on to the nonwoven (0.5 gsm). The nonwoven containing hot melt glue was then laminated with the tissue layer hot melt glue and SAP. This gave the bottom layer of the absorbent paper. Another layer was prepared by spraying hot melt glue (2.0 gsm) on to another tissue sheet (top layer) and then another type of SAP (130 gsm) was applied on to the tissue layer. This gave second layer of absorbent paper.

The first layer and second layer were then laminated together using hot melt glue (0.5 gsm) by passing through a compression rolls. This resulted in a complete absorbent paper. Absorbent paper consisted of two layers of superabsorbent polymers (SAP) one of which laid on top side and another laid on the bottom of the sheet. Both top and bottom SAP layers contained 130 grams per square meter (g/$m^2$). Both layers were glued with 0.5 g/$m^2$ hot melt adhesive on 50 g/$m^2$ air-thru-bond nonwoven material and were then sandwiched with two layers of 45 g/$m^2$ condensed tissue layers on the top and bottom using hot-melt glue applied to the surface at 2.0 g/$m^2$. Total hot-melt glue used was 2.5 g/$m^2$ for

both top and bottom layers.

The lamination sheet (hereunder called specimen) was cut to give 95 mm width and 400 mm length.

Different absorbent papers were produced and shown in the table 4.

Table 4. Absorbent paper diapers with different SAPs as top and bottom layer

| | Top layer | Bottom layer |
|---|---|---|
| * | 130 gsm Sanwet IM930NP | 130 gsm Aquakeep SA60SX-II, |
| | 130 gsm example 8 | 130 gsm Aquakeep SA60SX-II, |
| | 130 gsm example 9 | 130 gsm Aquakeep SA60SX-II, |
| *Reference Example | | |

Example 11

Preparation of diaper samples

A commercial tape diaper having 2-layered embossed nonwoven topsheet with 52 gsm. The diaper sample was cut from the backsheet in the middle. The original absorbent core was carefully removed. The diaper sample had no acquisition layer. Both spunbond nonwoven leg cuffs (left and right) had 35 mm height and two elastic strands. The distance between the tack-down leg cuff and the absorbency core for both sides was 10 mm width. Other materials remain the same giving an empty like diaper sachet.

The Lab laminated absorbent paper was cut using a scissor to give a dimension of 400 mm length and 95 mm width. This sheet of absorbent paper was inserted and placed into diaper sachet. The diaper sample was then carefully sealed by adhesive tape to form diaper sample for Lab testing.

Example 12

For the absorbent papers as descripted in example 10 the Water Pouring Time and Rewet are determined. The results are summarized in Table 5

Table 5. Water Pouring Time and Rewet Test results

| | Top layer | Bottom layer | Water pouring time / sec | Rewet / g |
|---|---|---|---|---|
| * | 130 gsm Sanwet IM930NP | 130 gsm Aquakeep SA60SX-II | 33.0 | 0.8 |
| | 130 gsm example 8 | 130 gsm Aquakeep SA60SX-II | 28.0 | 0.7 |
| | 130 gsm example 9 | 130 gsm Aquakeep SA60SX-II | 31.0 | 0.4 |
| *Reference Example | | | | |

The results show that the absorbent paper laminates with blending of agglomerated fine water-absorbent polymer particles and Hysorb®N7059 in the top layer and Aquakeep SA60SX-II in the bottom layer respectively showed a faster water pouring time and lower rewet compared to the reference example

Example 14

For the absorbent papers as descripted in example 10 $TAC_{AP,30\ min}$, $CRC_{AP,30\ min}$ and $TAC_{AP,1\ min}$ are determined. The results are summarized in the Table 6.

Table 6. 30 mins TAC/CRC results

| | Top layer | Bottom layer | $TAC_{AP30\ min}$ g/g | $CRC_{AP,30\ min}$ g/g |
|---|---|---|---|---|
| * | 130 gsm Sanwet IM930NP | 130 gsm Aquakeep SA60SX-II | 500.0 | 310.0 |
| | 130 gsm example 8 | 130 gsm Aquakeep SA60SX-II | 501.0 | 323.0 |

(continued)

| | Top layer | Bottom layer | $TAC_{AP30\ min}$ g/g | $CRC_{AP,30\ min}$ g/g |
|---|---|---|---|---|
| | 130 gsm example 9 | 130 gsm Aquakeep SA60SX-II | 522.0 | 346.0 |
| *Reference Example | | | | |

Absorbent papers with example 8 or example 9 in the top layer and Aquakeep SA60SX-II in the bottom layer show significant higher $TAC_{AP30\ min}$ and $CRC_{AP,30\ min}$ compared to the reference.

## Claims

1. A fluid-absorbent core (80)comprising at least two layers, an upper layer (91) and a lower layer (92), each layer comprising from 0 to 10% by weight fibrous material and from 90 to 100% by weight water-absorbent polymer particles, based on the sum of water-absorbent polymer particles and fibrous material,

   wherein the water-absorbent polymer particles within the upper layer (91) are a water absorbent mixture comprising at least 10% by weight of a first type of water-absorbent polymer particles, water-absorbent polymer particles G, with a vortex of less than 30 s, determined according to the method disclosed in the description and at least 50% by weight of a second type of water-absorbent polymer particles, water-absorbent polymer particles H, with a CRC of at least 25 g/g determined by the EDANA test method No. WSP 241.3 (11) wherein water-absorbent polymer particles G are obtained by agglomerating fine water-absorbing polymer particles by using a solution or suspension comprising

   a) 0,04 to 1,2 % by weight water-soluble or water-dispersible polymeric binders or a mixture thereof, based on the water-absorbent polymer particles,
   b) 20 to 70% by weight of water based on the water-absorbent polymer particles, and
   c) 5 to 20% by weight of a water-miscible organic solvent based on the water-absorbent polymer particles

   and wherein fine water-absorbing polymer particles having an average particle diameter of not larger than 300 μm.

2. A fluid-absorbent core (80) according to claims 1, wherein the water-absorbent polymer particles G obtainable by agglomerating non-surface post-crosslinked fine water-absorbent polymer particles, surface post-crosslinked fine water-absorbent polymer particles or a blend of non-surface post-crosslinked fine water-absorbent polymer particles and surface post-crosslinked fine water-absorbent polymer particles, drying, grinding, sieving and classification of the agglomerated water-absorbent polymer particles.

3. A fluid-absorbent core (80) according to claim2, wherein the ratio of the non-surface post-crosslinked fine water-absorbent polymer particles to the surface post-crosslinked fine water-absorbent polymer particles is at least 2 to 1 by weight.

4. A fluid-absorbent core (80) according to any of claim2 wherein the ratio of the non-surface post-crosslinked fine water-absorbent polymer particles to the surface post-crosslinked fine water-absorbent polymer particles is at least 3 to 1 by weight.

5. A fluid-absorbent core (80) according to any of claims 1 to 4, wherein the water-absorbent polymer particles G have a vortex of less than 25 s.

6. A fluid-absorbent core (80) according to any of claims 1 to 5, wherein the water-absorbent polymer particles H have a CRC of at least 30 g/g.

7. A fluid-absorbent core (80) according to any of claims 1 to 6, wherein the water-absorbent polymer particles G have a bulk density of 0.55 g/ml or lower, determined according to EDANA test method No. WSP 242.3 (11).

8. A fluid-absorbent core (80) according to any of claims 1 to 7, wherein within the lower layer (92) water-absorbent

polymer particles with a CRC of at least 30 g/g and/or a vortex of at least 30 s.

**9.** A fluid-absorbent core (80) according to any of claims 1 to 8, wherein the upper layer (91) comprises 100% by weight of water-absorbent polymer particles and/or wherein the lower layer (92) comprises 100% by weight of water-absorbent polymer particles.

**10.** A fluid-absorbent core (80) according to any of claims 1 to 9, wherein a nonwoven material (94) is sandwiched between the upper layer (91) and the lower layer (92).

**11.** A fluid absorbent article, comprising

(A) an upper liquid-pervious sheet (89),
(B) a lower liquid-impervious sheet (83),
(C) a fluid-absorbent core (80) according to claims 1 to 10
(D) an acquisition-distribution layer (70) between the upper liquid-pervious sheet (89) and the fluid-absorbent core (80),
(F) other optional components.

**12.** A fluid absorbent article according to claim 11 with water pouring time of less than 35 s and a rewet value of less than 0,8 g, determined according to the method disclosed in the description.

**13.** A fluid absorbent article according to claim 11 or 12 with a TAC $_{30\ min}$ of more than 500 g/g, determined according to the method disclosed in the description.

## Patentansprüche

**1.** Fluidabsorbierender Kern (80) umfassend wenigstens zwei Schichten, eine obere Schicht (91) und eine untere Schicht (92), wobei jede Schicht von 0 bis 10 Gew.-% Fasermaterial und von 90 bis 100 Gew.-% an wasserabsorbierenden Polymerpartikeln, bezogen auf die Summe von wasserabsorbierenden Polymerpartikeln und Fasermaterial, umfasst,

wobei die wasserabsorbierenden Polymerpartikel in der oberen Schicht (91) ein wasserabsorbierendes Gemisch sind, das wenigstens 10 Gew.-% an einem ersten Typ von wasserabsorbierenden Polymerpartikeln, wasserabsorbierende Polymerpartikel G, mit einem Vortex-Wert von weniger als 30 s, bestimmt gemäß dem in der Beschreibung offenbarten Verfahren, und wenigstens 50 Gew.-% an einem zweiten Typ von wasserabsorbierenden Polymerpartikeln, wasserabsorbierende Polymerpartikel H, mit einer CRC von wenigstens 25 g/g, bestimmt durch das EDANA-Prüfverfahren Nr. WSP 241.3 (11), umfasst, wobei wasserabsorbierende Polymerpartikel G durch Agglomeration von feinen wasserabsorbierenden Polymerpartikeln unter Verwendung einer Lösung oder Suspension erhalten sind, die umfasst:

a) 0,04 bis 1,2 Gew.-% an wasserlöslichen oder wasserdispergierbaren polymeren Bindemitteln oder einem Gemisch davon, bezogen auf die wasserabsorbierenden Polymerpartikel,
b) 20 bis 70 Gew.-% Wasser, bezogen auf die wasserabsorbierenden Polymerpartikel, und
c) 5 bis 20 Gew.-% an einem wassermischbaren organischen Lösungsmittel, bezogen auf die wasserabsorbierenden Polymerpartikel,

und wobei feine wasserabsorbierende Polymerpartikel einen mittleren Partikeldurchmesser von nicht größer als 300 $\mu$m aufweisen.

**2.** Fluidabsorbierender Kern (80) gemäß Anspruch 1, wobei die wasserabsorbierenden Polymerpartikel G erhältlich sind durch Agglomeration von nicht oberflächennachvernetzten feinen wasserabsorbierenden Polymerpartikeln, oberflächennachvernetzten feinen wasserabsorbierenden Polymerpartikeln oder einem Gemisch von nicht oberflächennachvernetzten feinen wasserabsorbierenden Polymerpartikeln und oberflächennachvernetzten feinen wasserabsorbierenden Polymerpartikeln, Trocknen, Mahlen, Sieben und Klassieren der agglomerierten wasserabsorbierenden Polymerpartikel.

**3.** Fluidabsorbierender Kern (80) gemäß Anspruch 2, wobei das Verhältnis der nicht oberflächennachvernetzten feinen

wasserabsorbierenden Polymerpartikel zu den oberflächennachvernetzten feinen wasserabsorbierenden Polymerpartikeln wenigstens 2 zu 1 nach Gewicht beträgt.

**4.** Fluidabsorbierender Kern (80) gemäß Anspruch 2, wobei das Verhältnis der nicht oberflächennachvernetzten feinen wasserabsorbierenden Polymerpartikel zu den oberflächennachvernetzten feinen wasserabsorbierenden Polymerpartikeln wenigstens 3 zu 1 nach Gewicht beträgt.

**5.** Fluidabsorbierender Kern (80) gemäß einem der Ansprüche 1 bis 4, wobei die wasserabsorbierenden Polymerpartikel G einen Vortex-Wert von weniger als 25 s aufweisen.

**6.** Fluidabsorbierender Kern (80) gemäß einem der Ansprüche 1 bis 5, wobei die wasserabsorbierenden Polymerpartikel H eine CRC von wenigstens 30 g/g aufweisen.

**7.** Fluidabsorbierender Kern (80) gemäß einem der Ansprüche 1 bis 6, wobei die wasserabsorbierenden Polymerpartikel G eine Schüttdichte von 0,55 g/ml oder niedriger, bestimmt gemäß dem EDANA-Prüfverfahren Nr. WSP 242.3 (11), aufweisen.

**8.** Fluidabsorbierender Kern (80) gemäß einem der Ansprüche 1 bis 7, wobei in der unteren Schicht (92) wasserabsorbierende Polymerpartikel eine CRC von wenigstens 30 g/g und/oder einen Vortex-Wert von wenigstens 30 s aufweisen.

**9.** Fluidabsorbierender Kern (80) gemäß einem der Ansprüche 1 bis 8, wobei die obere Schicht (91) 100 Gew.-% an wasserabsorbierenden Polymerpartikeln umfasst und/oder wobei die untere Schicht (92) 100 Gew.-% an wasserabsorbierenden Polymerpartikeln umfasst.

**10.** Fluidabsorbierender Kern (80) gemäß einem der Ansprüche 1 bis 9, wobei ein Vliesmaterial (94) zwischen der oberen Schicht (91) und der unteren Schicht (92) eingeschlossen ist.

**11.** Fluidabsorbierender Gegenstand, umfassend

(A) eine obere flüssigkeitsdurchlässige Lage (89),
(B) eine untere flüssigkeitsundurchlässige Lage (83),
(C) einen fluidabsorbierenden Kern (80) gemäß Ansprüchen 1 bis 10,
(D) eine Aufnahme-Verteilungs-Schicht (70) zwischen der oberen flüssigkeitsdurchlässigen Lage (89) und dem fluidabsorbierenden Kern (80),
(F) andere optionale Komponenten.

**12.** Fluidabsorbierender Gegenstand gemäß Anspruch 11 mit einer Wassergießzeit von weniger als 35 s und einem Wiederbenetzungswert von weniger als 0,8 g, bestimmt gemäß dem in der Beschreibung offenbarten Verfahren.

**13.** Fluidabsorbierender Gegenstand gemäß Anspruch 11 oder 12 mit einer $TAC_{30\ min}$ von mehr als 500 g/g, bestimmt gemäß dem in der Beschreibung offenbarten Verfahren.

## Revendications

**1.** Noyau absorbant les fluides (80) comprenant au moins deux couches, une couche supérieure (91) et une couche inférieure (92), chaque couche comprenant de 0 à 10 % en poids de matériau fibreux et de 90 à 100 % en poids de particules de polymère absorbant l'eau, sur la base de la somme des particules de polymère absorbant de l'eau et du matériau fibreux,

dans lequel les particules de polymère absorbant l'eau dans la couche supérieure (91) sont un mélange absorbant l'eau comprenant au moins 10 % en poids d'un premier type de particules de polymère absorbant l'eau, les particules de polymère absorbant l'eau G, avec un tourbillon de moins de 30 s, déterminé selon la méthode décrite dans la description et au moins 50 % en poids d'un deuxième type de particules de polymère absorbant l'eau, les particules de polymère absorbant l'eau H, ayant une CRC d'au moins 25 g/g déterminée par la méthode d'essai EDANA n° WSP 241.3 (11), les particules de polymère absorbant l'eau G étant obtenues par agglomération de particules fines de polymère absorbant l'eau au moyen d'une solution ou suspension comprenant

a) 0,04 à 1,2 % en poids de liants polymères solubles dans l'eau ou dispersibles dans l'eau ou un mélange de ceux-ci, sur la base des particules de polymère absorbant l'eau,
b) 20 à 70 % en poids d'eau sur la base des particules de polymère absorbant l'eau, et
c) 5 à 20 % en poids d'un solvant organique miscible dans l'eau sur la base des particules de polymère absorbant l'eau

et dans lequel les particules fines de polymère absorbant l'eau ayant un diamètre de particule moyen inférieur ou égal à 300 $\mu$m.

**2.** Noyau absorbant les fluides (80) selon la revendication 1, dans lequel les particules de polymère absorbant l'eau G peuvent être obtenues par agglomération de particules fines de polymère absorbant l'eau non post-réticulées en surface, de particules fines de polymère absorbant l'eau post-réticulées en surface ou d'un mélange de particules fines de polymère absorbant l'eau non post-réticulées en surface et de particules fines de polymère absorbant l'eau post-réticulées en surface, séchage, broyage, tamisage et classification des particules de polymère absorbant l'eau agglomérées.

**3.** Noyau absorbant les fluides (80) selon la revendication 2, dans lequel le rapport des particules fines de polymère absorbant l'eau non post-réticulées en surface aux particules fines de polymère absorbant l'eau post-réticulées en surface est d'au moins 2 à 1 en poids.

**4.** Noyau absorbant les fluides (80) selon la revendication 2, dans lequel le rapport des particules fines de polymère absorbant l'eau non post-réticulées en surface aux particules fines de polymère absorbant l'eau post-réticulées en surface est d'au moins 3 à 1 en poids.

**5.** Noyau absorbant les fluides (80) selon l'une quelconque des revendications 1 à 4, dans lequel les particules de polymère absorbant l'eau G ont un tourbillon inférieur à 25 s.

**6.** Noyau absorbant les fluides (80) selon l'une quelconque des revendications 1 à 5, dans lequel les particules de polymère absorbant l'eau H ont une CRC d'au moins 30 g/g.

**7.** Noyau absorbant les fluides (80) selon l'une quelconque des revendications 1 à 6, dans lequel les particules de polymère absorbant l'eau G ont une masse volumique apparente de 0,55 g/ml ou moins, déterminée selon la méthode d'essai EDANA n° WSP 242.3 (11).

**8.** Noyau absorbant les fluides (80) selon l'une quelconque des revendications 1 à 7, dans lequel, dans la couche inférieure (92), les particules de polymère absorbant l'eau ont une CRC d'au moins 30 g/g et/ou un tourbillon d'au moins 30 s.

**9.** Noyau absorbant les fluides (80) selon l'une quelconque des revendications 1 à 8, dans lequel la couche supérieure (91) comprend 100 % en poids de particules de polymère absorbant l'eau et/ou dans lequel la couche inférieure (92) comprend 100 % en poids de particules de polymère absorbant l'eau.

**10.** Noyau absorbant les fluides (80) selon l'une quelconque des revendications 1 à 9, dans lequel un matériau non-tissé (94) est intercalé entre la couche supérieure (91) et la couche inférieure (92).

**11.** Article absorbant les fluides, comprenant

(A) une feuille supérieure perméable aux liquides (89),
(B) une feuille inférieure imperméable aux liquides (83),
(C) un noyau absorbant les fluides (80) selon les revendications 1 à 10
(D) une couche d'acquisition-distribution (70) entre la feuille supérieure perméable aux liquides (89) et le noyau absorbant les fluides (80),
(F) d'autres composants facultatifs.

**12.** Article absorbant les fluides selon la revendication 11 avec un temps de coulée d'eau inférieur à 35 s et une valeur de réhumidification inférieure à 0,8 g, déterminée selon le procédé décrit dans la description.

**13.** Article absorbant les fluides selon la revendication 11 ou 12 avec une $TAC_{30\ min}$ supérieure à 500 g/g, déterminée

selon le procédé décrit dans la description.

Fig. 1

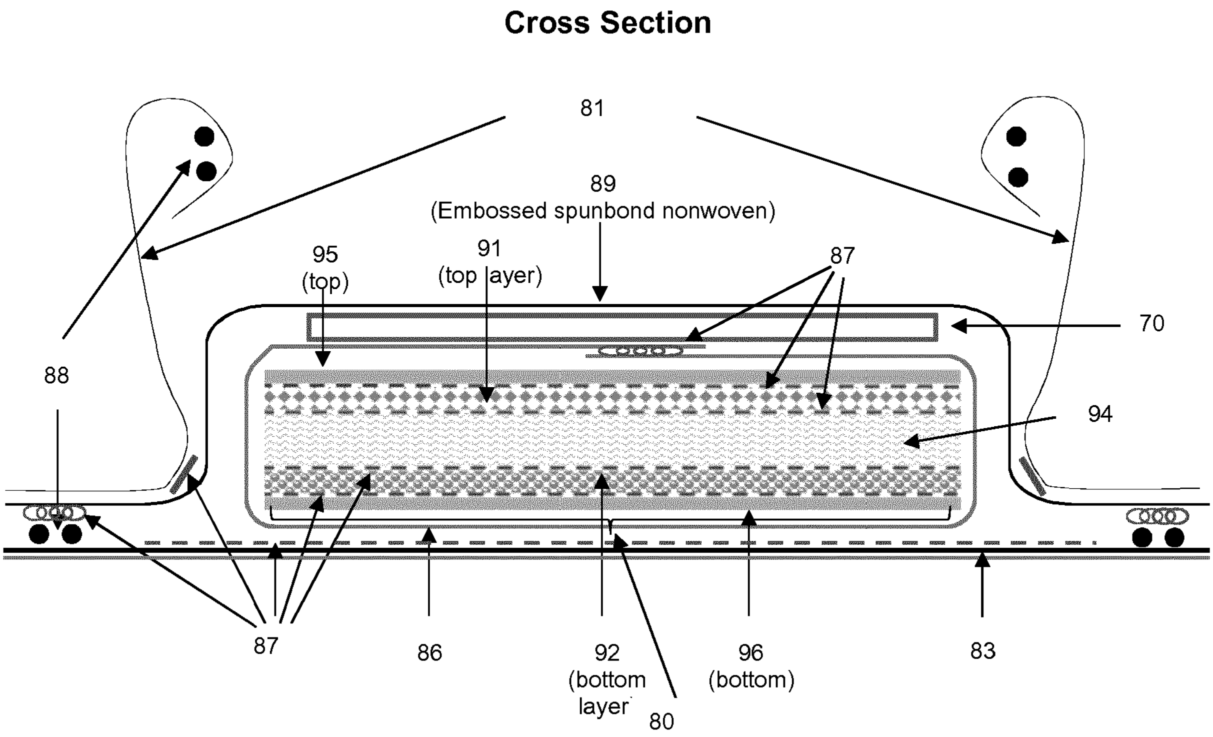
Cross Section
81
89
(Embossed spunbond nonwoven)
95
(top)
91
(top layer)
87
70
88
94
87
86
92
(bottom
layer
80
96
(bottom)
83

Fig. 2

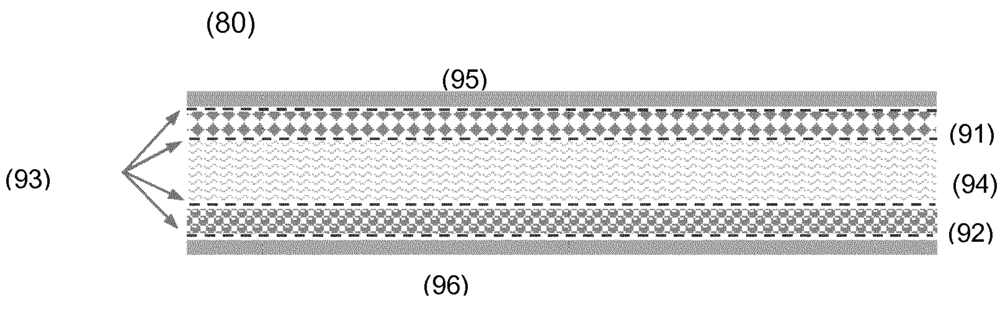
(80)
(95)
(93)
(91)
(94)
(92)
(96)

Fig. 3

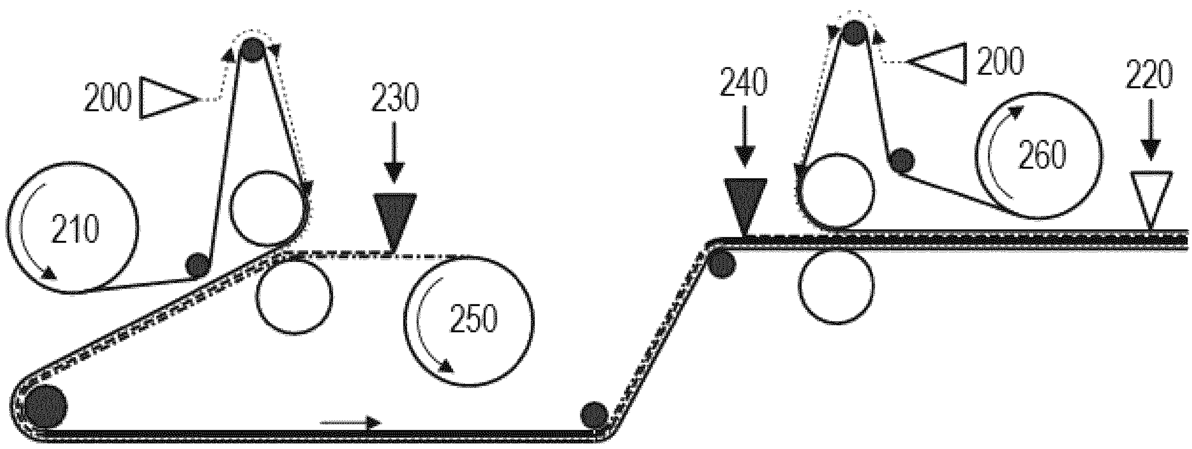

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 445619 A2
- DE 19846413 A1
- WO 0138402 A1
- EP 955086 A2
- DE 3825366 A1
- US 6241928 B
- EP 457660 A1
- WO 2008040715 A2
- WO 2008052971 A1
- WO 2008069639 A1
- WO 2008086976 A1
- WO 2014079694 A
- WO 2015028327 A
- WO 2015028158 A
- EP 2944376 A1
- WO 2011086842 A
- EP 2565031 A1
- EP 2668936 A1
- US 20170281425 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- WO 2001038402 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- EP 0999238 A1
- DE 19854573 A1
- DE 19854574 A1
- DE 10204937 A1
- DE 10334584 A1
- EP 1199327 A2
- WO 200331482 A1
- DE 3713601 A1
- WO 2012045705 A1
- WO 2004024816 A1
- EP 2301499 A1
- EP 2314264 A1
- EP 2387981 A1
- EP 2486901 A1
- EP 2524679 A1
- EP 2524680 A1
- US 6972011 B
- US 20110162989 A
- US 20110270204 A
- WO 2010004894 A1
- WO 2010004895 A1
- WO 2010076857 A1
- WO 2010082373 A1
- WO 2010118409 A1
- WO 2010133529 A2
- WO 2010143635 A1
- WO 2011084981 A1
- WO 2011086841 A1
- WO 2011086842 A1
- WO 2011086843 A1
- WO 2011086844 A1
- WO 2011117997 A1
- WO 2011136087 A1
- WO 2012048879 A1
- WO 2012052173 A1
- WO 2012052172 A1


### Non-patent literature cited in the description


- **F.L. BUCHHOLZ ; A.T. GRAHAM.** Modern Superabsorbent Polymer Technology. Wiley-VCH, 1998, 252-258

- **F.L. BUCHHOLZ ; A.T. GRAHAM.** Modern Superabsorbent Polymer Technology. Wiley-VCH, 1998, 71-103
- **ROBERT F. GOULD.** Contact angle, wettability and adhesion. American Chemical Society, 1964